# EUROPEAN PATENT APPLICATION

(11) **EP 2 837 325 A1**
(43) Date of publication of application: **18.02.2015**
(21) Application number: 13775623.5
(22) Date of filing: 10.04.2013
(51) Int. Cl.: A61B 1/04, A61B 1/00, G02B 23/24

(54) **ENDOSCOPE DEVICE AND ENDOSCOPE SYSTEM**

(30) Priority: 11.04.2012 JP 2012090332
(71) Applicant: Olympus Corporation, Shibuya-ku Tokyo 151-0072 (JP)
(72) Inventor: KOBAYASHI Naruyasu, Tokyo 151-0072 (JP); AZUMA Motoo, Tokyo 151-0072 (JP)
(74) Representative: Gunzelmann, Rainer
(86) International application number: PCT/JP2013/060811
(87) International publication number: WO 2013/154130

(57) **Abstract**

The present invention is capable of setting a program corresponding to each programmable device for the programmable devices. This endoscope device and endoscope system are provided with: programmable devices performing processing in accordance with programs; and a rewrite control unit which transmits unique information which is specific to the programmable devices to an external storage device retaining the programs, receives the program corresponding to the unique information from the external storage device, and sets the received programs to the programmable devices.

## Description

### TECHNICAL FIELD

The present invention relates to an endoscope device and an endoscope system having a programmable device that performs a processing according to a program.

Priority is claimed on Japanese Patent Application No. 2012-090332, filed April 11, 2012, the content of which is incorporated herein by reference.

### BACKGROUND ART

An endoscope device uses a programmable device on which a program for performing a processing such as image processing can be installed. By updating the program that is installed on the programmable device, it is possible to improve the functionality and performance of the endoscope device. Patent Document 1 discloses an endoscope device using a method for updating the program that is installed on the programmable device. In Patent Document 1, the firmware of the endoscope device is connected to an external storage device such as a PC, the version of the program is confirmed, and then the program is updated.

### CITATION LIST

### PATENT DOCUMENT

### [Patent Document 1]

Japanese Unexamined Patent Application, First Publication No. 2000-245682

### DISCLOSURE OF INVENTION

### PROBLEMS TO BE SOLVED BY THE INVENTION

In the prior art, only the version of the program itself is referred to when the program is updated. Viewing the information for identifying the programmable device in the endoscope device is not referenced.

In some cases, in the endoscope device, a plurality of scopes and a plurality of image processors (hereinafter referred to as processor) are used, the combination of the scopes and the image processors being changed so as to suit the type of the diagnosis. Further, each product of the scopes and the processors may be used beyond the generation of product. The combination of the scopes and the processors is wide-ranging.

However, when generations of the scope and the processor that are used vary widely, there is a case where a device of a previous generation cannot respond to a device of the latest generation. That is, the combination of the devices cannot be employed. For example, if an endoscope of the latest generation is connected to a processor of an old generation, image processing may not be performed properly. This problem occurs because the scope and the processor cannot treat devices of the same generation. Therefore, both of the scope and the processor need to be updated and have the latest program.

The present invention is made in view of the above problems. The present invention aims to provide an endoscope device and an endoscope system in which a program corresponding to each programmable device is installed on the programmable device.

### MEANS FOR SOLVING THE PROBLEMS

According to a first aspect of the present invention, an endoscope device communicates with an external storage device, the external storage device storing a plurality of programs corresponding to a plurality of programmable devices. The endoscope device includes: a programmable device that performs a process in accordance with a program; and a rewrite control unit that transmits to the external storage device unique information specific to the programmable device, the rewrite control unit receiving from the external storage device a program corresponding to the unique information, the rewrite control unit installing the program, which has been received, on the programmable device.

Also, according to a second aspect of the present invention, in the endoscope device according to the first aspect, the unique information may be a type and an ID of the programmable device, and the rewrite control unit may include a unique information storage unit having a nonvolatile storage medium that stores the ID.

Also, according to a third aspect of the present invention, in the endoscope device according to the second aspect, the rewrite control unit may further include a communication unit that transmits to the external storage device the ID that has been stored in the unique information storage unit.

Also, according to a fourth aspect of the present invention, in the endoscope device according to the third aspect, the communication unit may further receive from the external storage device the program corresponding to the ID, and the rewrite control unit may further include an update information storage unit having a storage medium that stores the program that has been received from the external storage device.

Also, according to a fifth aspect of the present invention, in the endoscope device according to the fourth aspect, the rewrite control unit may further include a program setting unit that reads the program, which corresponds to the ID that has been stored in the unique information storage unit, from the update information storage unit, the program setting unit installing the program, which has been read, on the programmable device.

Also, according to a sixth aspect of the present invention, in the endoscope device according to the fifth aspect, the communication unit may further receive version information of the program from the external storage device, the unique information storage unit may store the program and the version information, which have been installed on the programmable device, the program and the version information being associated with the ID, and the update information storage unit may store the program and the version information, which have been received, the program and the version information being associated with the ID.

Also, according to a seventh aspect of the present invention, in the endoscope device according to the sixth aspect, the program setting unit may compare the version information, which has been stored in the unique information storage unit, and the version information, which has been stored in the update information storage unit, that are associated with a same ID, read from the update information storage unit or the unique information storage unit the program associated with a newer version information, and install the program, which has been read, on the programmable device.

Also, according to an eighth aspect of the present invention, in the endoscope device according to the seventh aspect, if the version information stored in the update information storage unit is newer than the version information stored in the unique information storage unit, the program setting unit may further rewrite the program and the version information, which have been stored in the unique information storage unit, into the program and the version information that have been stored in the update information storage unit.

Also, according to a ninth aspect of the present invention, in the endoscope device according to the fifth aspect, if the communication unit is disconnected from the external storage device, the program setting unit may further read the program from the unique information storage unit and installs the program, which has been read, on the programmable device.

Also, according to a tenth aspect of the present invention, in the endoscope device according to the fifth aspect, the communication unit may receive the program, which corresponds to the ID, and the parameter information, which is specific to the programmable device, from the external storage device, the update information storage unit may store the program and the parameter information, which have been received from the external storage device, the program and the parameter information being associated with the ID, the program setting unit may read the program, which corresponds to the ID that is stored in the unique information storage unit, from the update information storage unit, install the program, which has been read, on the programmable device, read the parameter information, which corresponds to the ID and stored in the unique information storage unit, from the update information storage unit, and install the parameter information, which has been read, on the programmable device.

Also, according to an eleventh aspect of the present invention, the endoscope device according to the sixth aspect may include a plurality of the programmable device. The unique information storage unit may store the program and the version information, the program and the version information being associated with the ID, for each of the plurality of the programmable devices.

Also, according to a twelfth aspect of the present invention, in the endoscope system according to the fifth aspect, the communication unit may further transmit to the external storage device the ID and the version information of the program corresponding to the ID.

Also, according to a thirteenth aspect of the present invention, an endoscope system includes an external storage device, which holds a plurality of programs corresponding to the plurality of programmable devices, and an endoscope device. The endoscope device includes: a programmable device that performs a process according to the program; and a rewrite control unit that transmits a type of the programmable device, an ID of the programmable device and the version information corresponding to the ID, receives the program corresponding to the ID from the external storage device, and installs the program, which has been received, on the programmable device. The rewrite control unit includes: a unique information storage unit having a nonvolatile storage medium that stores the ID; a first communication unit that transmits to the external storage device the ID, which has been stored in the unique information storage unit, and the version information of the program corresponding to the ID, the first communication unit receiving the program corresponding to the ID from the external storage device; an update information storage unit having a storage medium that stores the program that has been received from the external storage device; and a program setting unit that reads from the update information storage unit the program corresponding to the ID that has been stored in the unique information storage unit, the program setting unit installing the program, which has been read, on the programmable device. The external storage device includes: an information storage unit having a storage medium that stores the program and the version information, the program and the version information being associated with the ID; a second communication unit that receives the ID and the version information from the endoscope device, the second communication unit transmitting to the endoscope device the program, which corresponds to the ID that has been received from endoscope device, of the program that has been stored in the information storage unit; and a control unit that reads from the information storage unit the version information, which corresponds to the ID that has been received from the endoscope device, so as to compare with the version information, which has been received from the endoscope device, the control unit determining whether or not to transmit the program, which has been stored in the information storage unit, to the endoscope device based on a result of the comparing.

### EFFECTS OF THE INVENTION

According to the endoscope device and the endoscope system described above, the endoscope device transmits to the external storage device, which stores a plurality of programs corresponding to a plurality of programmable devices, unique information that is unique to the programmable device in the endoscope device itself.

By receiving the program of the programmable device corresponding to the unique information from the external storage device, the program corresponding to each programmable device can be installed on the programmable device.

### BRIEF DESCRIPTION OF THE DRAWINGS

FIG 1 is a block diagram illustrating a configuration of an endoscope system in accordance with a first preferred embodiment of the present invention.
FIG. 2 is a block diagram illustrating a configuration of an update information management unit and an external storage device in an endoscope device in accordance with the first preferred embodiment of the present invention.
FIG 3 is a reference diagram illustrating a configuration of a data stored in a unique information storage unit and an update information storage unit in the endoscope device in accordance with the first preferred embodiment of the present invention.
FIG 4 is a flowchart illustrating a procedure of an operation of the endoscope device in accordance with the first preferred embodiment of the present invention.
FIG 5 is a flowchart illustrating the procedure of the operation of the endoscope device in accordance with the first preferred embodiment of the present invention.
FIG. 6 is a block diagram illustrating a correspondence between the configuration and steps of the operation of the endoscope device in accordance with the first preferred embodiment of the present invention.
FIG. 7 is a flowchart illustrating the procedure of the operation of the endoscope device in accordance with the first preferred embodiment of the present invention.
FIG. 8 is a flowchart illustrating a procedure of an operation of the endoscope device in accordance with a second preferred embodiment of the present invention.
FIG. 9 is a flowchart illustrating the procedure of the operation of the endoscope device in accordance with the second preferred embodiment of the present invention.
FIG. 10 is a block diagram illustrating a correspondence between the configuration and steps of the operation of the endoscope device in accordance with the second preferred embodiment of the present invention.
FIG. 11 is a flowchart illustrating a procedure of an operation of the endoscope device in accordance with a third preferred embodiment of the present invention.
FIG. 12 is a flowchart illustrating the procedure of the operation of the endoscope device in accordance with the third preferred embodiment of the present invention.
FIG. 13 is a block diagram illustrating a correspondence between the configuration and steps of the operation of the endoscope device in accordance with the third preferred embodiment of the present invention.
FIG. 14 is a block diagram illustrating a configuration of an endoscope system in accordance with a fourth preferred embodiment of the present invention.
FIG. 15 is a reference diagram illustrating a configuration of a data stored in a unique information storage unit and an update information storage unit in the endoscope device in accordance with the fourth preferred embodiment of the present invention.
FIG. 16 is a flowchart illustrating a procedure of an operation of the endoscope device in accordance with the fourth preferred embodiment of the present invention.
FIG. 17 is a flowchart illustrating a procedure of an operation of the endoscope device in accordance with the fourth preferred embodiment of the present invention.
FIG 18 is a block diagram illustrating a configuration of an endoscope system in accordance with a fifth preferred embodiment of the present invention.
FIG. 19 is a reference diagram illustrating a configuration of a data stored in a unique information storage unit and an update information storage unit in the endoscope device in accordance with the fifth preferred embodiment of the present invention.
FIG. 20 is a flowchart illustrating a procedure of an operation of the endoscope device in accordance with the fifth preferred embodiment of the present invention.
FIG 21 is a flowchart illustrating the procedure of the operation of the endoscope device in accordance with the fifth preferred embodiment of the present invention.
FIG 22 is a flowchart illustrating the procedure of the operation of the endoscope device in accordance with the fifth preferred embodiment of the present invention.
FIG. 23 is a flowchart illustrating the procedure of the operation of the endoscope device in accordance with the fifth preferred embodiment of the present invention.
FIG 24 is a reference diagram illustrating a configuration of a data stored in an update information storage unit in the endoscope device in accordance with the fifth preferred embodiment of the present invention.
FIG 25 is a reference diagram illustrating a configuration of a data stored in an update information storage unit in the endoscope device in accordance with the fifth preferred embodiment of the present invention.
FIG. 26 is a flowchart illustrating a procedure of an operation of the endoscope device in accordance with a sixth preferred embodiment of the present invention.
FIG. 27 is a block diagram illustrating a correspondence between the configuration and steps of the operation of the endoscope device in accordance with the sixth preferred embodiment of the present invention.

### PREFERRED EMBODIMENTS FOR CARRYING OUT THE INVENTION

Hereinafter, preferred embodiments of the present invention will be described with reference to the drawings.

### (First Preferred Embodiment)

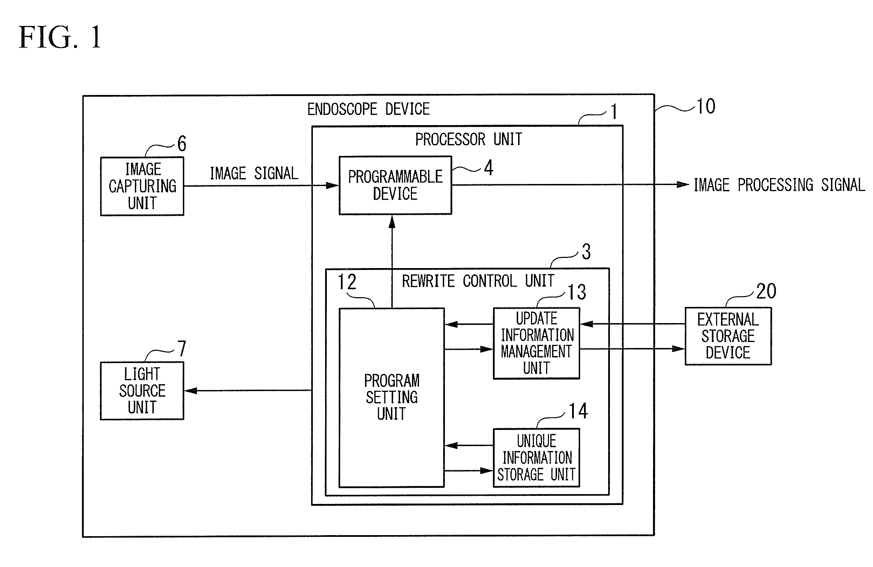

First, a first preferred embodiment of the present invention will be described. FIG. 1 shows a configuration of an endoscope system in accordance with the present embodiment. As shown in FIG. 1, the endoscope system of the present embodiment includes an endoscope device 10 and an external storage device 20.

The endoscope device 10 includes a processor unit 1, an image capturing unit 6, and a light source unit 7. The processor unit 1 performs image processing. The image capturing unit 6 converts the light input through the optical system into an electrical signal, and outputs it as an image signal. The light source unit 7 generates the light irradiating the subject.

The processor unit 1 includes a rewrite control unit 3 and a programmable device 4. The programmable device 4 includes a rewritable circuit that performs image processing function based on the program. Also, the programmable device 4 performs an arbitrary processing on the image signal output from the image capturing unit 6, and outputs it as an image processing signal. The rewrite control unit 3 controls rewriting of the programmable device 4.

The rewrite control unit 3 includes a program setting unit 12, an update information management unit 13, and a unique information storage unit 14. The unique information storage unit 14 includes a nonvolatile storage medium that stores an ID for identifying the type of the endoscope device 10 and the individual programmable device 4 and a program to be installed on the programmable device 4. The update information management unit 13 communicates with the external storage device 20, and stores a program or the like received from the external storage device 20. The program setting unit 12 performs programming (installs) on the programmable device 4 the program that is determined as the latest program among the programs stored in the unique information storage unit 14 and the programs stored in the update information management unit 13.

The external storage device 20 is connected to the endoscope device 10 via a network such as a LAN. The external storage device 20 manages the program or the like of each of a plurality of programmable devices including the programmable device 4, and transmits to the endoscope device 10 the program or the like corresponding to the ID transmitted from the endoscope device 10.

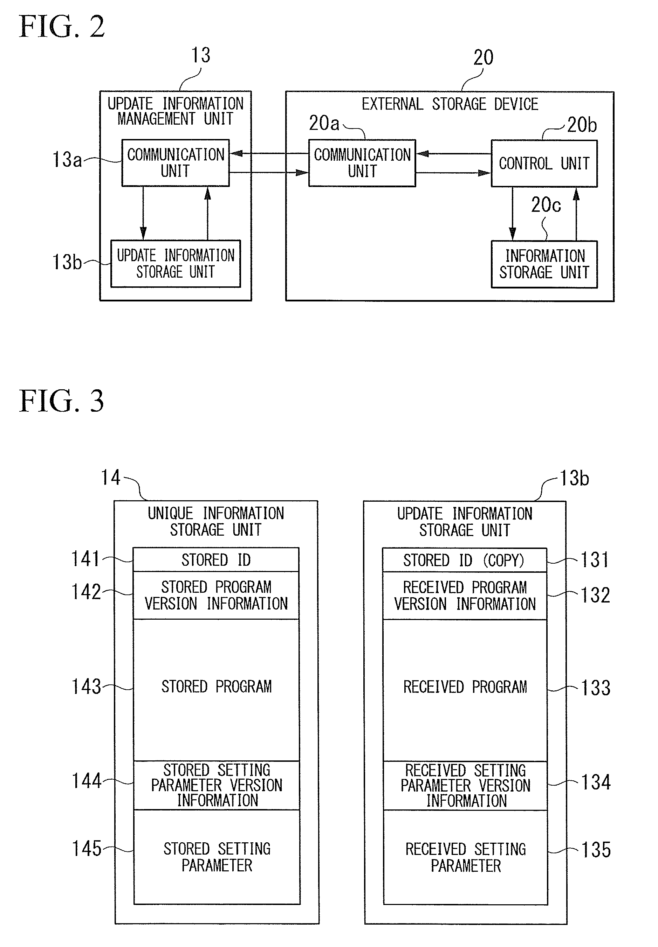

FIG. 2 shows the structure of the update information management unit 13 and the external storage device 20 in the endoscope device 10. The update information management unit 13 includes the communication unit 13a and the update information storage unit 13b. The communication unit 13a performs a communication with the external storage device 20. The update information storage unit 13b includes a volatile storage medium for storing the program or the like received from the external storage device 20. The external storage device 20 includes a communication unit 20a, a control unit 20b, and an information storage unit 20c. The communication unit 20a performs a communication with the endoscope device 10. The control portion 20b performs control to transmit to the endoscope device 10 the program corresponding to the ID received from the endoscope assembly 10. The information storage unit 20c includes a nonvolatile storage medium storing a program or the like of each of the plurality of programmable devices.

FIG. 3 shows the structure of a data stored in the unique information storage unit 14 and the update information storage unit 13b. The unique information storage unit 14 stores a stored ID 141, a stored program version information 142, a stored program 143, a stored setting parameter version information 144, and a stored setting parameters 145, which are associated with each other. The stored ID 141 is information unique to the type of the endoscope device 10 and the individual programmable device 4. The stored program version information 142 is information indicating the version of the stored program 143. The stored program 143 is a program that includes a code that defines the circuit configuration of the programmable device 4. The stored setting parameter version information 144 is information indicating the version of the stored setting parameter 145. The stored setting parameter 145 is a parameter (for example, image size or a filter coefficients required for the image processing) that defines the operation of the programmable device 4.

The update information storage unit 13b stores a stored ID 131, a received program version information 132, a received program 133, a received setting parameter version information 134, and a received setting parameters 135, which are associated with each other. Each data stored in the update information storage unit 13b corresponds to each data stored in the unique information storage unit 14.

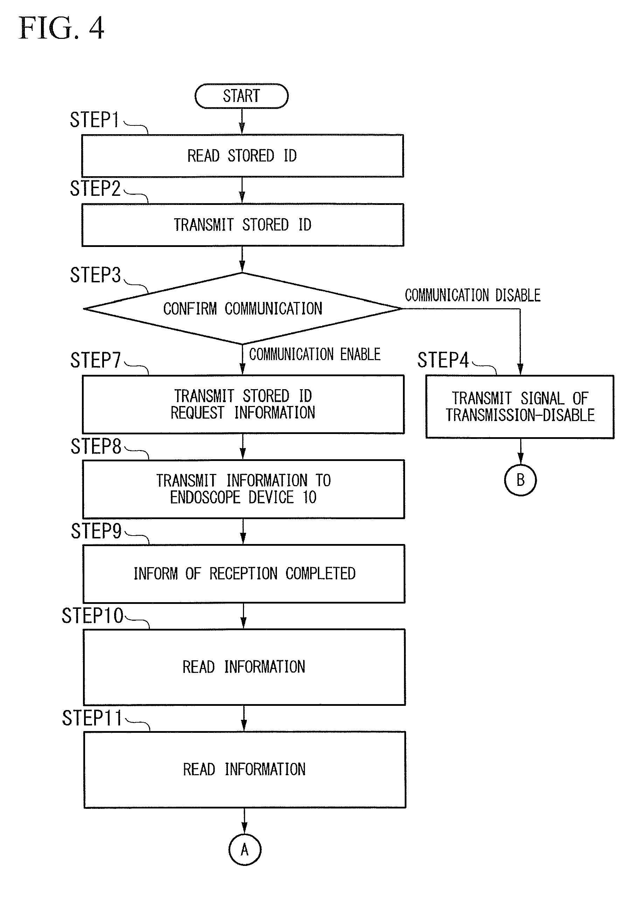

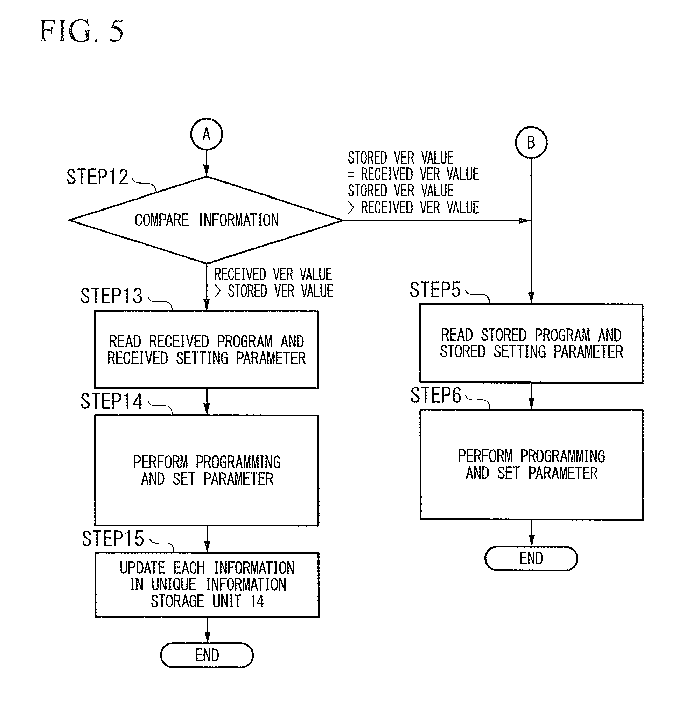

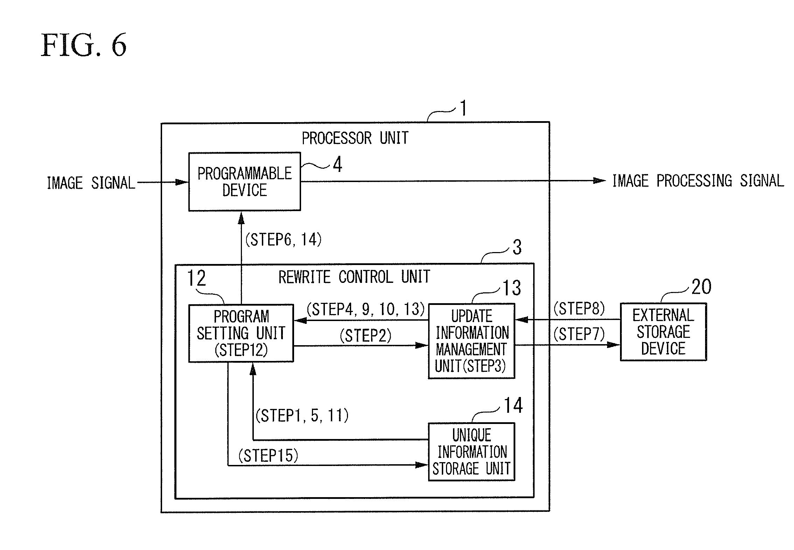

Next, the operation of the endoscope device 10 in accordance with the present embodiment will be described. FIGS. 4 and 5 show the procedure of the operation of the endoscope device 10. FIG. 6 shows the relationship between the steps of FIGS. 4 and 5 and the configuration of the configuration shown in FIG. 1 that relates to the operation of the present embodiment. Hereinafter, the operation of the endoscope device 10 will be described with reference to FIGS. 4 to 6.

### (STEP 1)

The program setting unit 12 reads the stored ID 141 from the unique information storage unit 14.

### (STEP 2)

The program setting unit 12 transmits the stored ID 141 that has been read to the update information management unit 13.

### (STEP 3)

The communication unit 13a of the update information management unit 13 has a function of determining whether it is possible to communicate with the external storage device 20. The communication unit 13a determines whether it is possible to communicate with the external storage device 20, by using a technique confirming the presence or absence of the connection of the LAN cable that is publicly known or a technique confirming the response of the external storage device 20.

Based on the result of the determination by the communication unit 13a of the update information management unit 13, the processing will branch as follows. If the communication with the external storage device 20 is impossible, then the processing of STEPS 4 to 6 is performed. If the communication is possible, then the processing of STEPS 5 to 15 is performed.

Operations of the case when the communication unit 13a of the update information management unit 13 determines that the communication with the external storage device 20 is impossible will be as follows.

### (STEP 4)

The communication unit 13a of the update information management unit 13 transmits the signal of transmission-disable to the program setting unit 12.

### (STEP 5)

If receiving the signal of transmission-disable, the program setting unit 12 reads the stored program 143 and the stored setting parameters 145 from the unique information storage unit 14.

### (STEP 6)

The program setting unit 12 performs programming and setting of parameters for the programmable devices 4 by using the stored program 143 and the stored setting parameters 145 that have been read.

As described above, rewriting to the programmable device 4 has been completed.

Operations of the case when the communication unit 13a of the update information management unit 13 determines that the communication with the external storage device 20 is possible will be as follows.

### (STEP 7)

The communication unit 13a of the update information management unit 13 stores the ID 141, which has been received from the program setting unit 12, once as the stored ID 131 in the update information storage unit 13b. The communication unit 13a of the update information management unit 13 transmits the stored ID 131 that has been stored to the external storage device 20, and requests information about each of the latest program, the latest program version information, the latest setting parameter, and the latest setting parameter version information that correspond to the stored ID 131.

### (STEP 8)

The external storage device 20 stores in the information storage unit 20c the program and the setting parameter corresponding to each of the plurality of types and the plurality of programmable devices. The communication unit 20a of the external storage device 20 transmits the stored ID 131, which has been received, to the control unit 20b. The control unit 20b specifies the type and the programmable device 4 based on the stored ID 131. The control portion 20b reads from the information storage unit 20c the latest program, the latest program version information, the latest setting parameter, and the latest setting parameter version information corresponding to the type and the programmable device 4, which have been specified, and transmits them to the endoscope device 10 via the communication unit 20a.

### (STEP 9)

The communication unit 13a of the update information management unit 13 stores temporarily in the update information storage unit 13b the received program as the received program 133, the received program version information as the received program version information 132, the received setting parameters as the received setting parameters 135, and the received setting parameter version information as the setting parameter version information 134. Thereafter, the communication unit 13a of the update information management unit 13 notifies the program setting unit 12 that the reception from the external storage device 20 has been completed.

### (STEP 10)

If receiving the notification of reception completion, the program setting unit 12 reads the received program version information 132 and the received parameter setting version information 134 from the update information storage unit 13b of the update information management unit 13.

### (STEP 11)

The program setting unit 12 reads the stored program version information 142 and the stored setting parameter version information 145 and from the unique information storage unit 14.

### (STEP 12)

The program setting unit 12 compares the received program version information 132, which has been read from the update information storage unit 13b of the update information management unit 13, with the stored program version information 142, which has been read from the unique information storage unit 14, and compares the received setting parameter version information 134, which has been read from the update information storage unit 13b of the update information management unit 13, with the stored setting parameter version information 145, which has been read from the unique information storage unit 14, to confirm whether or not each information coincides with each other.

Based on the result of the comparison by the program setting unit 12, the processing will branch as follows. If both version information coincides with each other, or if it is determined that the version information read from the unique information storage unit 14 is newer than the version information read from the update information storage unit 13b of the update information management unit 13, then the processing of STEPS 5 to 6 is carried out, and the rewriting process is completed. Further, if it is determined that the version information read from the update information storage unit 13b of the update information management unit 13 is newer than the version information read from the unique information storage unit 14, then the processing of STEPS 13 to 15 is performed.

### (STEP 13)

If the program setting unit 12 determines that the value of the received program version information 132 and the received setting parameter version information 134 do not coincide with the value of the stored program version information 142 and the stored setting parameter version information 145 (the value of the received program version information 132 and the received setting parameter version information 134 are larger than the value of the stored program version information 142 and the stored setting parameter version information 145), then the program setting unit 12 reads the received program 133 and the received setting parameter 135 from the update information storage unit 13b of the update information management unit 13.

### (STEP 14)

The program setting unit 12 performs programming and setting of parameters for the programmable device 4 by using the received program 133 and the received setting parameter 135.

### (STEP 15)

The program setting unit 12 reads from the update information storage unit 13b of the update information management unit 13 each information of the received program version information 132, the received program 133, the received setting parameter version information 134, and the received setting parameter 135, and updates (overwrites) each information of corresponding stored program version information 142, stored program 143, stored setting parameter version information 144, and stored setting parameter 145 that have been stored in the unique information storage unit 14. After the update is completed, rewriting to a programmable device 4 is completed.

In general, when comparing each version information, a method of judging based on the magnitude of the values are used. It is possible to determine the version of the largest value as the version of the latest version.

As described above, each time using an endoscope device 10, by properly obtaining from the external storage device 20 the latest program and the latest setting parameter corresponding to the type and the programmable device 4, and by programming and setting of the setting parameter of the programmable devices 4, it is possible to use the endoscope device 10 in the latest condition. In the present embodiment, even when types of the endoscope device are the same and the programmable devices mounted to the endoscope devices are different, the program corresponding to each programmable device can be installed on the programmable device by using the unique information that can specify the programmable device.

Further, it is possible to obtain the latest program and the latest setting parameter by performing a communication between the external storage device 20, which manages / stores the latest program, and the endoscope device 10. Furthermore, if communication with the external storage device 20 is available, then the information in the unique information storage unit 14 is updated. Thus, even in a state in which the communication with the external storage device 20 cannot be performed, the endoscope device 10 can be operated at least by programming and setting of parameter of the programmable device 4 using the latest program and the setting parameter that are previously obtained.

Further, it is possible to store the information of the ID in the unique information storage unit 14, which has a nonvolatile storage medium, even without a power supply by storing the type and the ID, which is information of the programmable device 4.

Further, it is possible to start the subsequent processing after the reception has completed by temporarily storing the program or the like, which is received from the external storage device 20, in the update information storage unit 13b. If the processing of the program or the like is performed in parallel with the reception of the program or the like, the reception may be interrupted due to the influence of the communication state, and the processing cannot be performed normally by loss of the information. Regarding the above, it is possible to avoid a situation in which the processing cannot be performed normally by starting the subsequent processing after the end of the reception.

Further, exactly one set of the program, the setting parameter and the version information is stored in the unique information storage unit 14 and the update information storage unit 13b. Thus, it is possible to confirm whether or not the version is latest or not, and programming and updating (overwriting) of the setting parameter can be performed rightly. Further, the endoscope device 10 can be used in the latest state by comparing the version information and selecting and installing the program and the setting parameter corresponding to the latest version information on the programmable device 4.

Further, if the version of the data stored in the update information storage unit 13b is newer than the version of the data stored in the unique information storage unit 14, then the data stored in the unique information storage unit 14 is updated (overwritten) with the data stored in the update information storage unit 13b. Thereby, it is possible to make data, which is stored in the unique information storage unit 14, correspond to the latest version. If communication with the external storage device 20 is unavailable, only the program of the unique information storage unit 14 is available. Thereby, the program used to rewrite the programmable device 4 can be selected from the program of the unique information storage unit 14 and the program of the update information storage unit 13b, which has been received from the external storage device 20, based on whether or not the communication with the external storage device 20 is available. Thus, even if the communication with the external storage device 20 is unavailable, the programmable device 4 can be rewritten by using the program stored in the unique information storage unit 14.

Also, various modifications are possible in the present embodiment. In the present embodiment, the program setting unit 12 installs the received program 133 and the received setting parameter 135 of the update information storage unit 13b directly on the programmable device 4. However, these information are only temporarily stored in the update information storage unit 13b. It is not possible to deal with the case such as when the power is turned off before completing the transmission of the information to the programmable device 4.

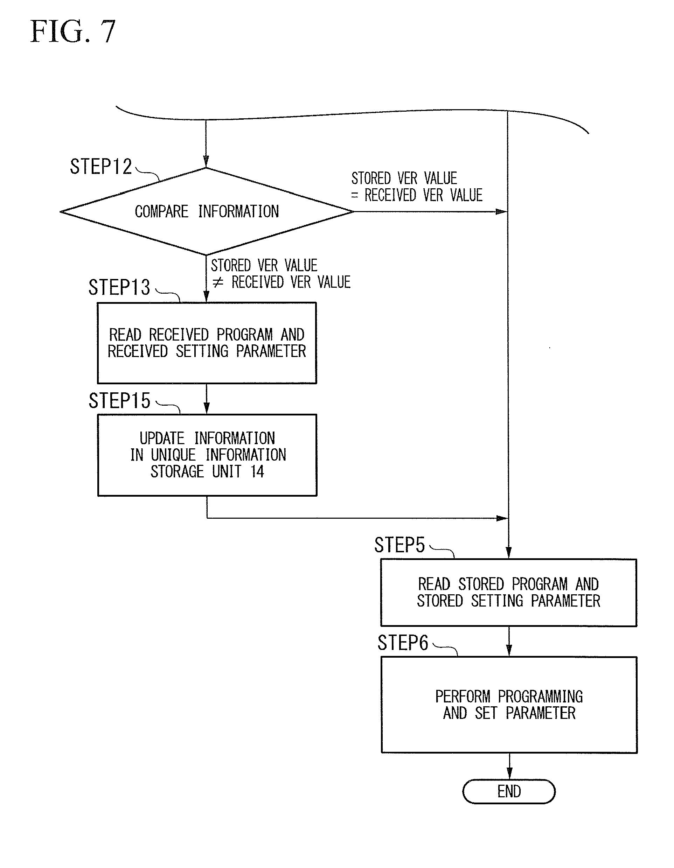

In this case, the program setting unit 12 first updates (overwrites) the stored program 143 and the stored setting parameter 145 of the unique information storage unit 14 by reading the received program 133 and the received setting parameter 135 from the update information storage unit 13b. Then, it is also possible to read the stored program 143 and the stored setting parameter 145 from the unique information storage unit 14, and installs them on the programmable device 4. In this case, the order of the processing in FIGS. 4 and 5 is changed as shown in FIG. 7. That is, the processing is performed in the order of STEPS 13, 14, 5, and 6.

The programmable device of the present embodiment is a device such as an FPGA that can form a circuit according to a predetermined program. However, it may be a device (CPU, for example) including a circuit for performing a predetermined operation in accordance with a program including an executable code in the machine language.

Further, the storage medium constituting the unique information storage unit 14 is any non-volatile storage medium, and FlashROM, HDD, SSD-like, or the like may be used.

Furthermore, the external storage device 20 is any device that can select the program and the parameter corresponding to the stored ID 131 that has been received from a plurality of programs and a plurality of parameters. It may be one or more personal computers or a large computer.

### (Second Preferred Embodiment)

Next, a second preferred embodiment of the present invention will be described. The configuration of the present embodiment is the same as the configuration of the first preferred embodiment. In this embodiment, the procedure of the processing is different from the procedure of processing of the first preferred embodiment. First, the program setting unit 12 obtains each version information of the program and the setting parameter from the external storage device 20, and compares them with each version information stored in the unique information storage unit 14. After that, obtaining of the program and the setting parameter is started. In the present embodiment, description thereof is omitted for the same reference numerals with respect to those having the same functions as those in the first preferred embodiment.

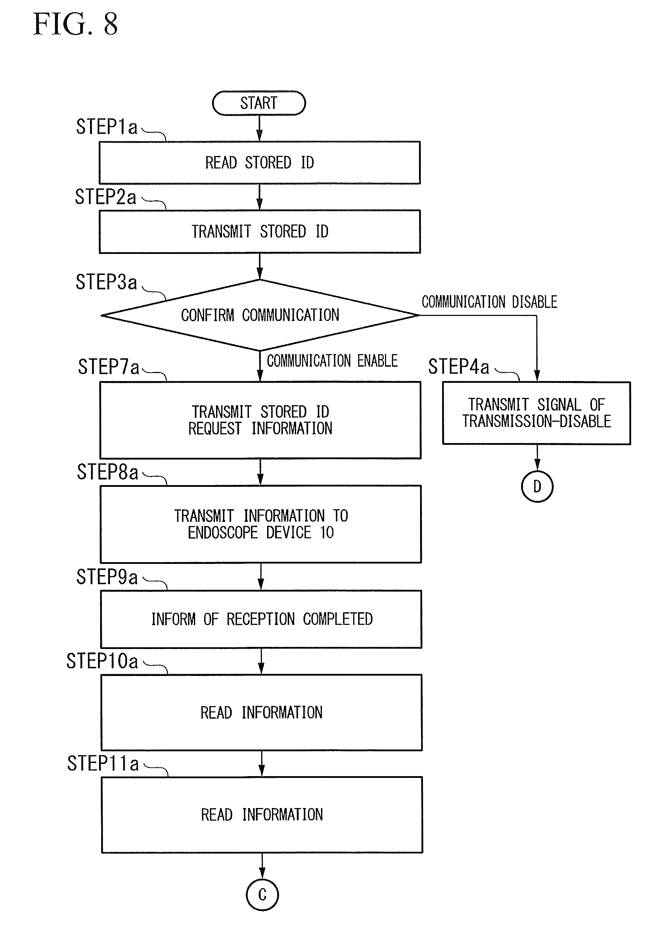

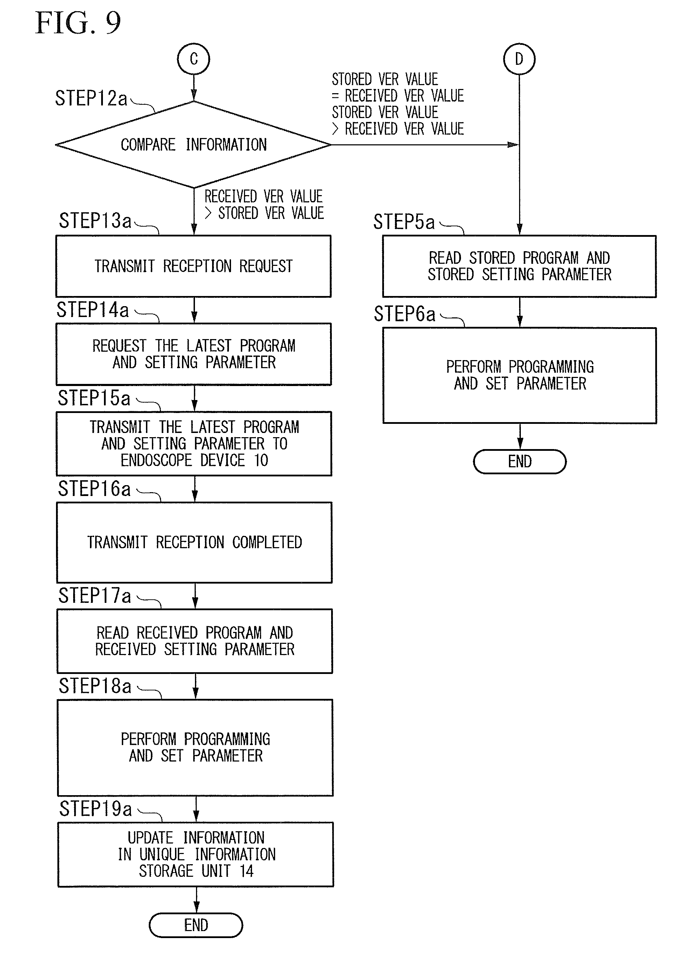

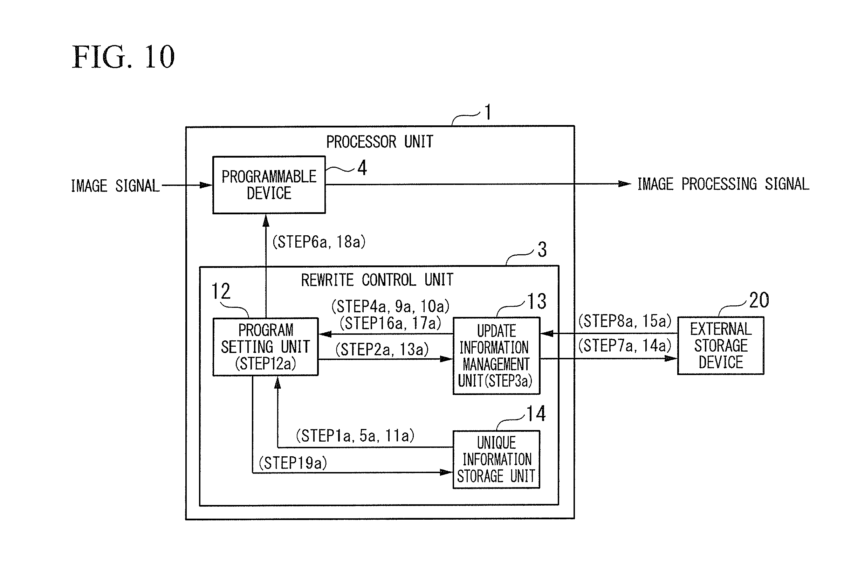

Next, the operation of the endoscope device 10 of the present embodiment will be described. FIGS. 8 and 9 show the procedure of the operation of the endoscope device 10. FIG. 10 shows the relationship between each step of FIGS. 8 and 9 and the configuration related to the operation of the present embodiment of the configuration shown in FIG 1. Hereinafter, a description of the operation of the endoscope device 10 will be described with reference to FIGS. 8 to 10.

### (STEPS 1a to 6a)

Descriptions will be omitted because the same processing is performed as the processing in STEPS 1 to 6 shown in the first preferred embodiment.

### (STEP 7a)

The communication unit 13a of the update information management unit 13 transmits the stored ID 131 to the external storage device 20 similarly to the first preferred embodiment, and requests the latest program version information and the latest setting parameter version information corresponding to the stored ID 131 that has been transmitted.

### (STEP 8a)

The communication unit 20a of the external storage device 20 transmits the stored ID 131, which has been received, to the control unit 20b. The control unit 20b specifies the type and the programmable device 4 based on the stored ID 131. The control unit 20b reads from the information storage unit 20c the latest program version information and the latest setting parameter version information corresponding to the type and the programmable device 4 that have been specified, and transmits them to the endoscope device 10 via the communication unit 20a .

### (STEPS 9a to 12a)

Descriptions will be omitted because the same processing is performed as the processing in STEPS 9 to 12 shown in the first preferred embodiment.

### (STEP 13a)

If the program setting unit 12 determines that the value of the received program version information 132 and the received setting parameter version information 134 do not coincide with the value of the stored program version information 142 and the stored setting parameter version information 145 (the value of the received program version information 132 and the received setting parameter version information 134 are larger than the value of the stored program version information 142 and the stored setting parameter version information 145), then the program setting unit 12 requests the latest program and the latest setting parameter from the communication unit 13a of the update information management unit 13.

### (STEP 14a)

If receiving a request for the program and the setting parameter from the program setting unit 12, then the communication unit 13a of the update information management unit 13 transmits again the stored ID 131 to the external storage device 20 and requests the latest program and the latest setting parameter.

### (STEP 15a)

The communication unit 20a of the external storage device 20 transmits the stored ID 131 that has been received to the control unit 20b. The control unit 20b specifies the type and the programmable device 4 based on the stored ID 131. The control unit 20b reads the latest program and the setting parameter corresponding to the type and the programmable device 4 that have been specified from the information storage unit 20c, and transmits them to the endoscope device 10 via the communication unit 20a.

### (STEP 16a)

The communication unit 13a of the update information management unit 13 makes the received program as the received program 133 and the received setting parameter as the received setting parameter 135, and temporarily stores them in the update information storage unit 13b. Thereafter, the communication unit 13a of the update information management unit 13 notifies the program setting unit 12 that the reception from the external storage device 20 is completed.

### (STEPS 17a to 19a)

Descriptions will be omitted because the same processing is performed as STEPS 13 to 15 shown in the first preferred embodiment.

As described above, by obtaining the version information and program / setting parameter by dividing in two from the external storage device 20, it is possible to quickly receive the version information that is relatively small amount of data. Further, if the version information stored in the unique information storage unit 14 and the version information stored in the update information storage unit 13b by receiving from the external storage device 20 coincide with each other, then it is not necessary to receive the program and it is possible to reduce unnecessary load on the network.

Various modifications are possible in the present embodiment as well. For example, as in the first preferred embodiment, the program setting unit 12 reads the received program 133 and the received setting parameters 135 from the update information storage unit 13b, and updates (overwrites) the stored program 143 and the stored setting parameter 145 of the unique information storage unit 14. Then, a method of reading the stored program 143 and the stored setting parameter 145 from the unique information storage unit 14 and installing them on the programmable device 4 is also possible. It is also possible to perform various modifications described in the first preferred embodiment besides this.

### (Third Preferred Embodiment)

Next, a third preferred embodiment of the present invention will be described. The configuration of the present embodiment is the same as the configuration of the first preferred embodiment and the second preferred embodiment. The present embodiment is different in entities for comparing the ID and the version and the data for communicating.

In the first preferred embodiment and the second preferred embodiment, the program setting unit 12 compares each version information of the program and the setting parameter. The program and the setting parameter are obtained from the storage unit of the endoscope device 10 or the external storage device 20 based on the result of the comparing. Thereby, programming and setting of parameters are performed. In the present embodiment, the external storage device 20 receives the ID and each version information stored in the update information storage unit 13b from the endoscope device 10.

Then, the external storage device 20 compares each version information corresponding to the ID stored in the external storage device 20 itself with the version information that has been received. If determined that the version information does not coincide with each other, then the external storage device 20 transmits to the endoscope device 10 the program and the setting parameter stored in the external storage device 20 itself. Further, if determined that each version information coincide with each other, then the external storage device 20 does not transmit the program and the setting parameter to the endoscope device 10 and notifies the endoscope device 10 that each version information coincide with each other. In the present embodiment, description thereof will be omitted for the same reference numerals with respect to those having the same functions as those in the first preferred embodiment.

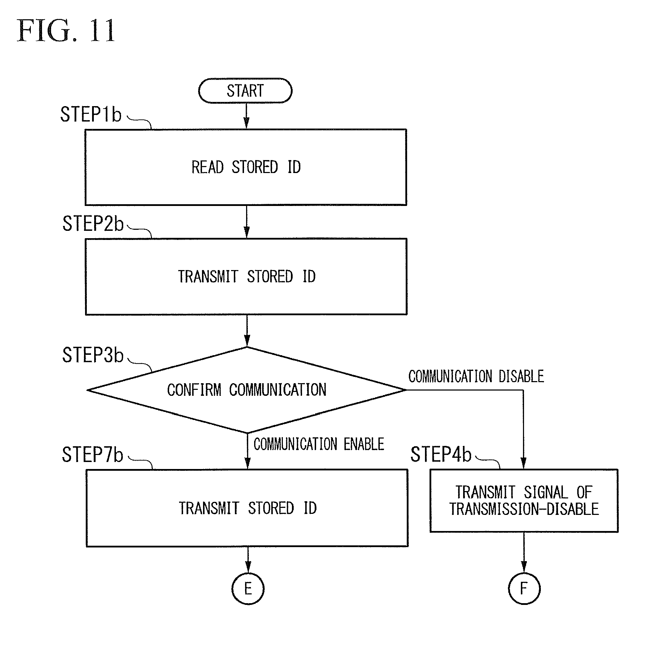

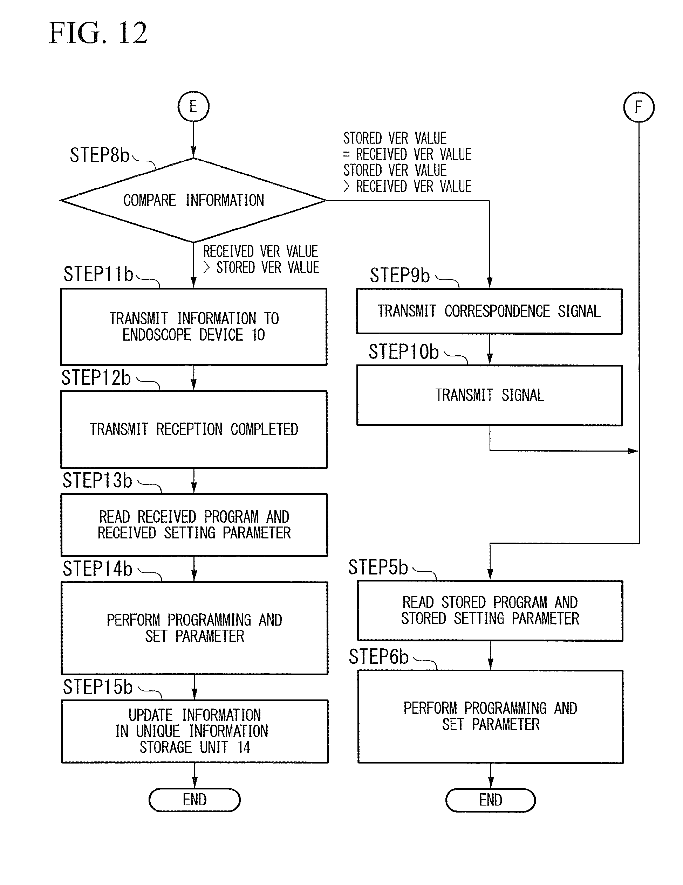

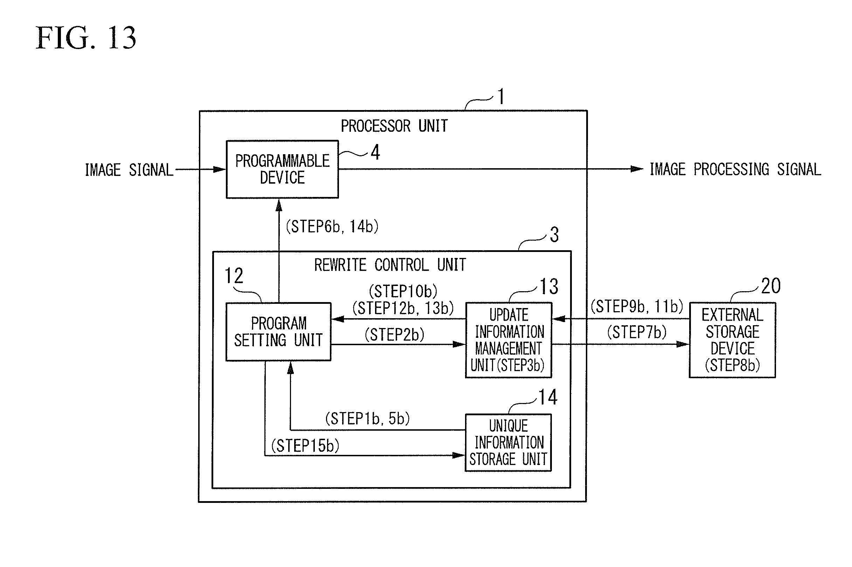

Next, the operation of the endoscope device 10 of the present embodiment will be described. FIGS. 11 and 12 are flowcharts illustrating the operation of the endoscope device 10. FIG. 13 shows the relationship between the steps of FIGS. 11 and 12 and the configuration related to the operation of the present embodiment of the configuration shown in FIG 1. Hereinafter, the operation of the endoscope device 10 will be described with reference to FIGS. 11 to 13.

### (STEP 1b)

The program setting unit 12 reads the stored ID 141, the stored program version information 142, and the stored setting parameter version information 144 from the unique information storage unit 14.

### (STEP 2b)

The program setting unit 12 transmits the stored ID 141, the stored program version information 142, and the stored setting parameter version information 144 that have been read to the update information management unit 13.

### (STEPS 3b to 6b)

Description will be omitted because the same processing is performed as in STEPS 3 to 6 shown in the first preferred embodiment.

### (STEP 7b)

The communication unit 13a of the update information management unit 13 transmits to the external storage device 20 the stored ID 141, the stored program version information 142, and the stored setting parameter version information 144, and requests for the latest program version information and the latest setting parameter version information.

### (STEP 8b)

The communication unit 20a of the external storage device 20 transmits to the control unit 20b the stored ID 141, the stored program version information 142, and the stored setting parameter version information 144 that have been received. The control unit 20b specifies the type and the programmable device 4 based on the stored ID 141. The control unit 20b reads from the information storage unit 20c the latest program version information and the latest setting parameter version information corresponding to the type and the programmable device 4 that have been specified. The stored program version information 142, which has been received, and the stored program version information, which has been read from the information storage unit 20c, are compared. Also, the stored setting parameter version information 144, which has been received, and the stored setting parameter version information, which has been read from the information storage unit 20c, are compared. Thereby, it is determined whether or not each information correspond with each other.

Based on the result of the comparison by the control unit 20b of the external storage device 20, the processing will branch as follows. If both version information coincides with each other or if it is determined that the version information read from the information storage unit 20c is newer than the version information received from the endoscope device 10, then the processing of STEPS 9b to 6b is performed.

### (STEP 9b)

The control unit 20b of the external storage device 20 sends a coincidence signal, which indicates that the version information coincide with each other, to the endoscope device 10 via the communication unit 20a.

### (STEP 10b)

If receiving the coincidence signal from the external storage device 20, then the communication unit 13a of the update information management unit 13 transmits a signal, which indicates that there is no update information, to the program setting unit 12.

If receiving a signal indicating that there is no update information, then the program setting unit 12 performs the processing of STEPS 5b to 6b.

If it is determined that the received version information is newer than the version information that has been read from the information storage unit 20c, then the processing of STEPS 11b to 15b is performed.

### (STEP 11b)

The control unit 20b of the external storage device 20 transmits the latest program, the latest program version information, the latest setting parameter, and the latest setting parameter version information corresponding to the stored ID 141 to the endoscope device 10 via the communication unit 20a

### (STEPS 12b to 15b)

Description will be omitted because the same processing is performed as the processing performed in STEPS 9 and 13 to 15 shown in the first preferred embodiment.

As described above, the external storage device 20 performs the comparison of the version information by the program setting unit 12 that has been performed in the first preferred embodiment and the second preferred embodiment. Accordingly, because the program setting unit 12 does not require a comparator circuit, it is possible to simplifying the configuration of the endoscope device 10. Also, as a result of the comparison of the version information, it is possible to perform the program settings for the programmable device 4 in the early stages of processing if there is no need to update the program and the setting parameter. In addition, since the endoscope device 10 transmits once to the external storage device 20 the information necessary to provide the comparison between the version information, the number of transmitting/receiving to/from the endoscope device 10 and the external storage device 20 can be reduced and the overhead time of processing by the communication can be reduced.

Various modifications are possible in the present embodiment as well. For example, as in the first preferred embodiment, the program setting unit 12 reads the received program 133 and the received setting parameter 135 from the update information storage unit 13b and updates (overwrites) the stored program 143 and the stored setting parameter 145 of the unique information storage unit 14. Then, a method of reading the stored program 143 and the stored setting parameter 145 from the unique information storage unit 14 and installing them on the programmable device 4 is also possible. It is also possible to perform various modifications described in the first preferred embodiment besides this.

### (Fourth Preferred Embodiment)

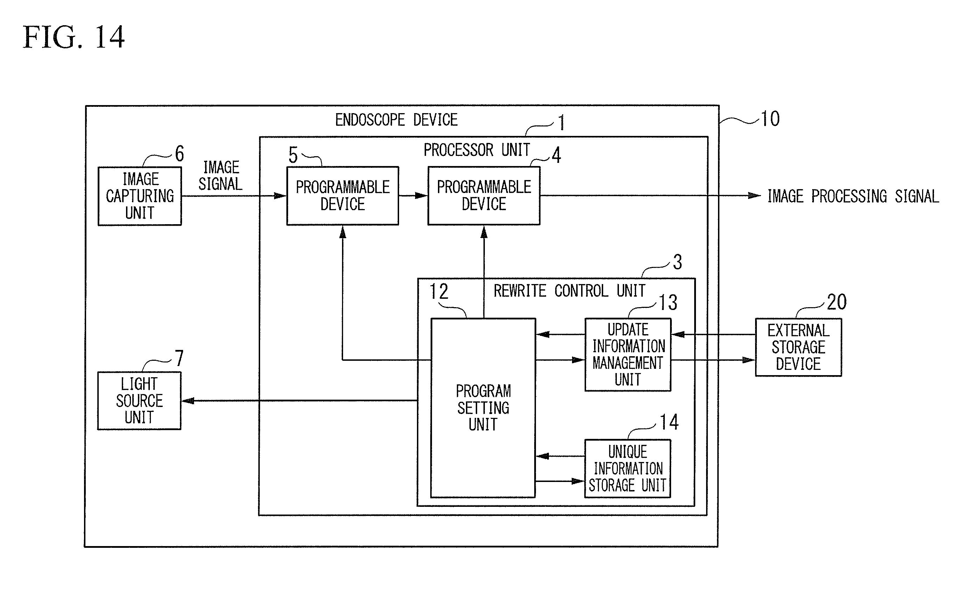

Next, a fourth preferred embodiment of the present invention will be described. FIG. 14 shows the configuration of an endoscope system according to the present embodiment. In the present embodiment, with respect to those having the same functions as those in the first preferred embodiment, description thereof is omitted for the same reference numerals.

The programmable device 5 is added to the processor unit 1 included in the endoscope device 10 of the present embodiment. The programmable device 5 has a rewritable circuit for performing the image processing function according to the program. The programmable device 5 performs an arbitrary processing on the image signal output from the image capturing unit 6, and outputs the processed image signal to the programmable device 4. The rewrite control unit 3 controls the rewriting of the programmable device 4 and the programmable device 5.

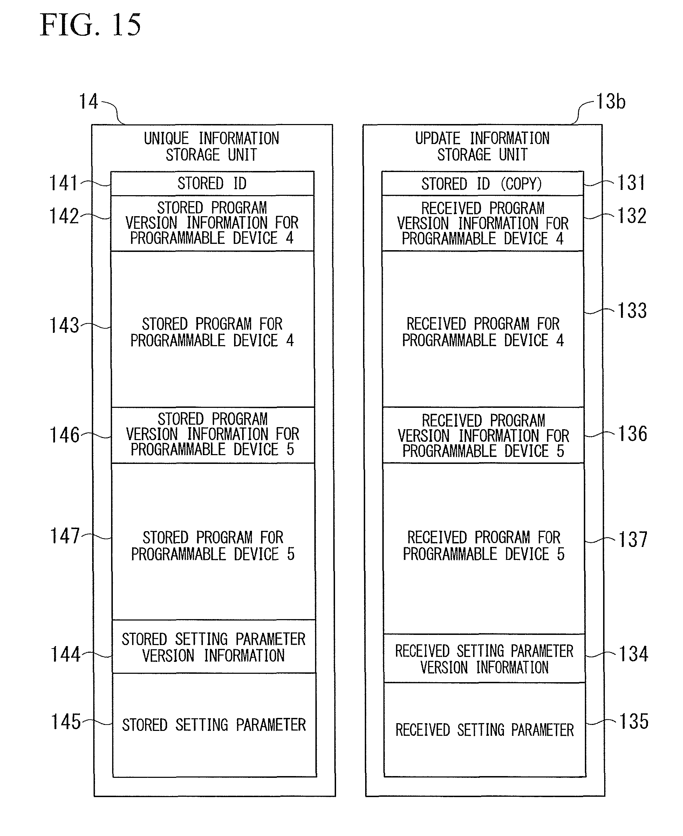

FIG 15 shows the structure of data stored in the unique information storage unit 14 and the update information management unit 13. The unique information storage unit 14 stores the stored ID 141, the stored program version information 142 for the programmable device 4, the stored program 143 for the programmable device 4, the stored program version information 146 for the programmable device 5, the stored program 147 for the programmable device 5, the stored setting parameter version information 144, and the stored setting parameter 145, which are associated with each other.

The update information storage unit 13b stores the stored ID 131, the received program version information 132 for the programmable device 4, the received program 133 for the programmable device 4, the received program version information 136 for the programmable device 5, the received program 137 for the programmable device 5, the received setting parameter version information 134, and the received setting parameter 135, which are associated with each other. Each data stored in the update information storage unit 13b corresponds to each data stored in the unique information storage unit 14.

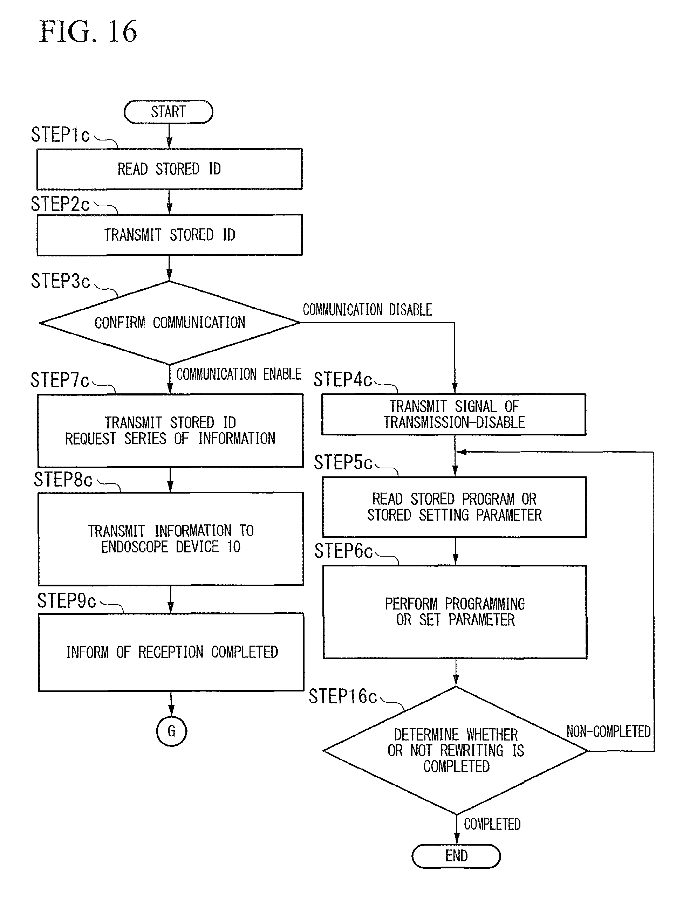

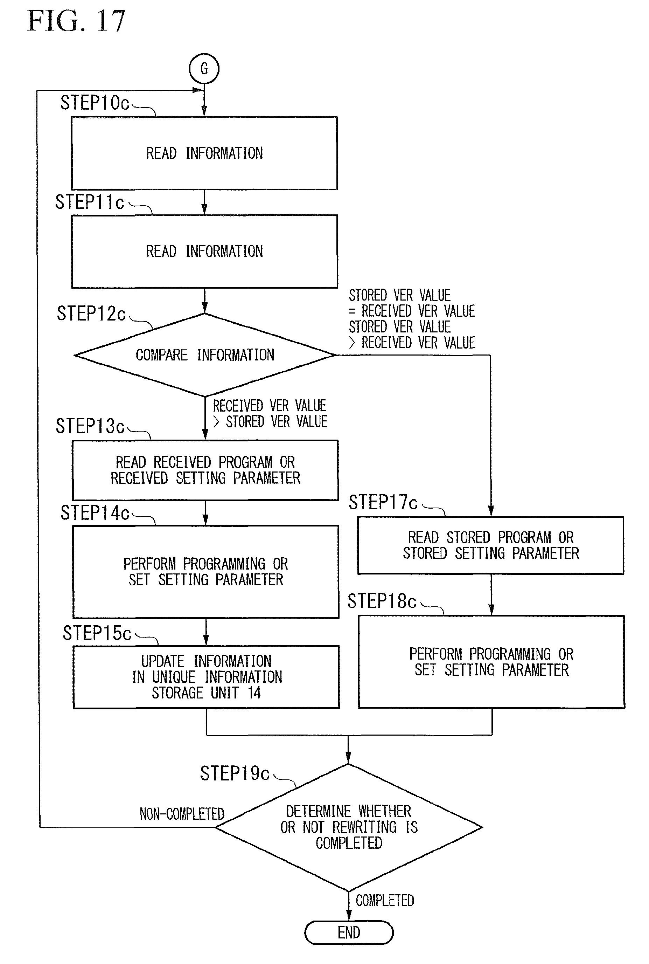

Next, the operation of the endoscope device 10 of the present embodiment will be described. FIGS. 16 and 17 show the procedure of the operation of the endoscope device 10. Hereinafter, the operation of the endoscope device 10 will be described with reference to FIGS. 16 and 17. In the present embodiment, after the programming for the programmable device 5 is performed, programing and setting of the setting parameter for the programmable device 4 are performed.

### (STEPS 1c to 7c)

Description will be omitted because the same processing is performed as those in STEPS 1 to 7 shown in the first preferred embodiment. However, if the programmable device 5 is to be processed, then the stored program 147 for the programmable device 5 is read from the unique information storage unit 14 by the processing of STEP 5c, and programming of the programmable device 5 using the stored program 147 by the processing of STEP 6c is performed.

### (STEP 16c)

The program setting unit 12 manages whether or not rewriting of all of the programmable devices has been completed. After the processing on the programmable device 5, the processing of STEPS 5c to 6c is performed again on the programmable device 4. In this case, the stored program 143 and the stored setting parameters 145 for the programmable device 4 are read from the unique information storage unit 14 by the processing of STEP 5c. By the processing of STEP 6c, programming of the programmable device 4 using the stored program 143 and settings of the settings parameter of the programmable device 4 using the parameter 145 are performed.

After updating of both the programmable device 4 and programmable device 5 is completed, the rewriting process is completed.

### (STEP 8c)

The communication unit 20a of the external storage device 20 transmits to the control unit 20b the stored ID 131 that has been received. The control unit 20b specifies the type, the programmable device 4, and the programmable device 5 based on the stored ID 131. The control unit 20b reads from the information storage unit 20c the latest program and the latest program version information corresponding the type, the programmable device 4, and the programmable device 5 that have been specified, and transmits them to the endoscope device 10 via the communication unit 20a.

### (STEP 9c)

The communication unit 13a of the update information management unit 13 stores temporary in the update information storage unit 13b the received program for the programmable device 4 as the received program 133, and the received program version information of the programmable device 4 as the received program version information 132. Further, the communication unit 13a temporarily stores in the update information storage unit 13b the received program for the programmable device 5 as the received program 137, and the received program version information for the programmable device 5 as the received program version information 137. Further, the communication unit 13a temporarily stores in the update information storage unit 13b the received setting parameter as the received setting parameter 135, and the received setting parameter version information as the received setting parameter version information 134. Thereafter, the communication unit 13a of the update information management unit 13 notifies the program setting unit 12 that the reception from the external storage device 20 is completed.

### (STEP 10c)

If receiving the notification of reception completion, the program setting unit 12 reads the received program version information 136 for the programmable device 5 from the update information storage unit 13b of the update information management unit 13.

### (STEP 11c)

The program setting unit 12 reads the stored program version information 146 for the programmable device 5 from the unique information storage unit 14.

### (STEP 12c)

The program setting unit 12 compares the received program version information 136 for the programmable device 5 read from the update information storage unit 13b of the update information management unit 13 with the stored program version information 146 for the programmable device 5 read from the unique information storage unit 14 to confirm whether or not each information correspond to each other.

Based on the result of the comparison by the program setting unit 12, the processing will branch as follows. The condition of the branch is the same as that in the first preferred embodiment. If both version information coincide with each other or if it is determined that the version information read from the unique information storage unit 14 is newer than the version information read from the update information storage unit 13b of the update information management unit 13, STEPS 17c to 18c is performed. Description will be omitted because the processing performed in STEPS 17c to 18c is the same as the processing performed in STEPS 5c to 6c.

### (STEP 13c)

If it is determined that the value of the received program version information 136 does not coincide with the value of the stored program version information 146 (the value of the received program version information 136 is greater than the value of the stored program version information 146), then the program setting unit 12 reads the receive program 137 from the update information storage unit 13b of the management unit 13.

### (STEP 14c)

The program setting unit 12 performs the program for the programmable device 5 by using the received program 137 that has been read.

### (STEP 15c)

The program setting unit 12 reads from the update information storage unit 13b of the update information management unit 13 information of each of the received program version information 136 and the received program 137, and updates (overwrites) each information of corresponding stored program version information 146 and stored program 147 that have been stored in the unique information storage unit 14.

### (STEP 19c)

The program setting unit 12 manages whether or not rewriting of all of the programmable devices has been completed. After the processing on the programmable device 5, the processing from STEP 5c is again performed on the programmable device 4.

If the programmable device 4 is processed, the stored program version information 142 and the stored setting parameter version information 144 for the programmable device 4 are read from the unique information storage unit 14 by the processing of STEP 10c. By the processing of the STEP 11 c, the received program version information 132 and the stored setting parameter version information 144 for the programmable device 4 are read from the update information storage unit 13b. By the process of STEP 12c, each version information is compared.

Also, if the programmable device 4 is to be processed, then the received program 133 and the received setting parameter 135 for the programmable device 4 are read out from the update information storage unit 13b by the processing of STEP 13c, and programming of the programmable device 4 using the received program 133 and setting of the setting parameter of the programmable device 4 using the received setting parameter 135 are performed by the processing of STEP 14c.

Also, if the programmable device 4 is processed, then the stored program 143 and the stored setting parameter 145 for the programmable device 4 are read from the unique information storage unit 14 by the processing of STEP 17c, and programming of the programmable device 4 using the stored program 143 and setting of the setting parameter of the programmable device 4 and the programmable device 5 using the stored setting parameter 145 are performed by the processing of STEP 18c. In addition, by the processing of STEP 15c, information of each of the received program version information 132, the received program 133, the received setting parameter version information 134, and the received setting parameter 135 is read from the update information storage unit 13b of the update information management unit 13. Information of each of corresponding stored program version information 142, stored program 143, stored setting parameter version information 144, received setting parameter 145 stored in the unique information update the storage unit 14 are updated (overwritten).

After updating both of the programmable device 4 and the programmable device 5 is completed, the rewriting processing is completed.

As described above, even if the endoscope device 10 has a plurality of programmable devices, it is possible to acquire the latest program and the latest setting parameter properly from the external storage device 20, and to perform programming and setting of the parameter of the programmable device.

In the present embodiment, a configuration having two programmable devices is described, it may be possible to have three or more programmable devices. In accordance with the number of programmable devices, information of the number of programmable devices may be stored in the ID. Further, the program version information and the program of each programmable device are stored in the unique information storage unit 14 and the update information storage unit 13b. Further, it is also possible to perform various modifications described in the first preferred embodiment.

### (Fifth Preferred Embodiment)

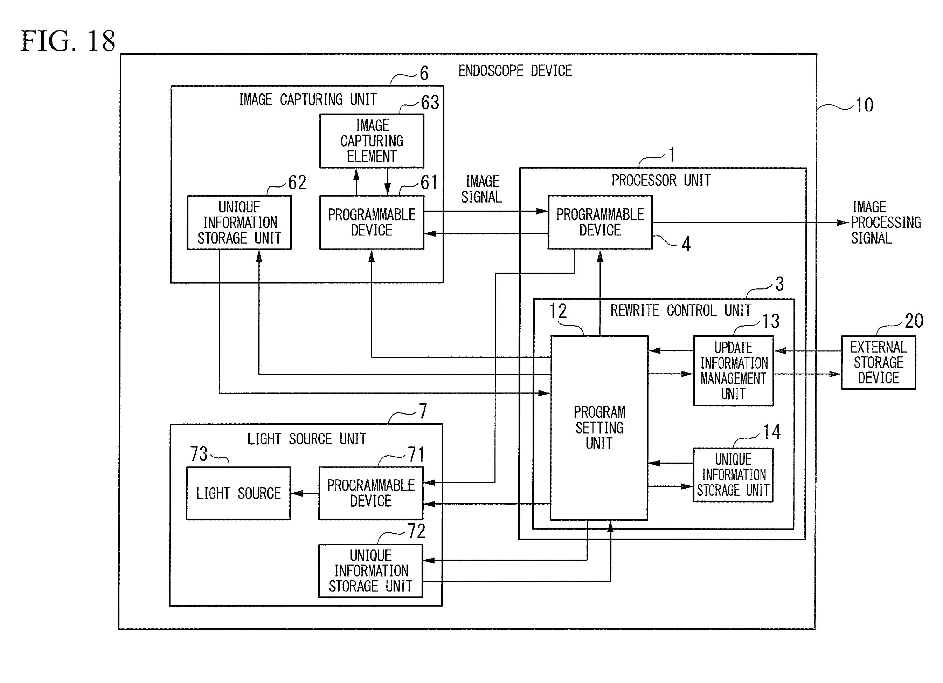

Next, a fifth preferred embodiment of the present invention will be described. FIG. 18 shows the configuration of an endoscope system in accordance with the present embodiment. In the present embodiment, with respect to those having the same functions as those in the first preferred embodiment, description thereof is omitted for the same reference numerals.

The endoscope device 10 of the present embodiment includes an image capturing unit 6 having a programmable device 6, the unique information storage unit 62, and an image capturing element 63. The programmable device 61 includes a rewritable circuit for control and image processing of the image capturing unit 6 in accordance with the program. The unique information storage unit 62 includes a nonvolatile storage medium for storing a program or the like used for the programming of the programmable device 61. The image capturing element 63 converts the light, which is input through the optical system, into an electrical signal to generate an image signal.

Also, the endoscope device 10 of the present embodiment includes a light source unit 7 having a programmable device 71, a unique information storage unit 72, and a light source 73. The programmable device 71 has a rewritable circuit for controlling the light source unit 7 according to the program. The unique information storage unit 72 includes a nonvolatile storage medium for storing a program or the like used for the programming of the programmable device 71. The light source 73 generates light that illuminates the subject. The rewrite the control unit 3 controls the rewriting of the programmable device 4, the programmable device 71, and the programmable device 61.

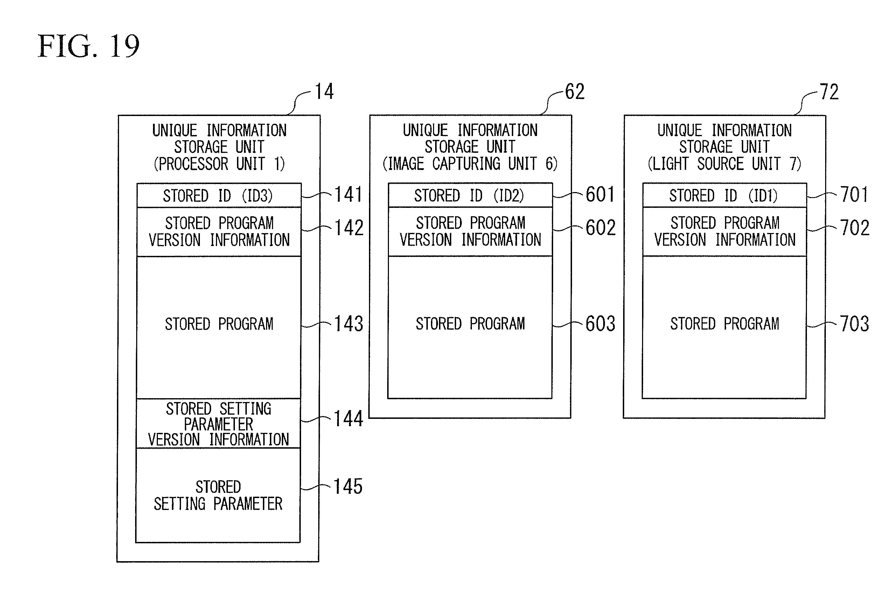

FIG. 19 shows the configuration of data stored in the unique information storage unit 14, the unique information storage unit 62, and the unique information storage unit 72. The unique information storage unit 14 stores a stored ID 141 (ID 3), a stored program version information 142, a stored program 143, a stored setting parameter version information 144, and a stored setting parameters 145, which are associated with each other. Each data stored in the unique information storage unit 14 is the same as the data stored in the unique information storage unit 14 of the first preferred embodiment. However, the stored setting parameter 145 of the present embodiment is a parameter that defines the operation of the programmable device 4. This parameter includes the parameter defining the operation of the programmable device 4 outputting a signal to the programmable device 61 and the programmable device 71. The signal output to the programmable device 61 by the programmable device 4 is a synchronous signal, for example. Further, the signal output to the programmable device 71 by the programmable device 4 is a signal for adjusting the brightness of the light source, for example.

The unique information storage unit 62 stores a stored ID 601 (ID 2), a stored program version information 602, and a stored program 603, which are associated with each other. The stored ID 601 is information specific to the type and an individual programmable device 61 of the endoscope device 10. The stored program version information 602 is information indicating the version of the stored program 603. The stored program 603 is a program that includes code that defines the circuit configuration of the programmable device 61.

The unique information storage unit 72 stores the stored ID 701 (ID 1), a stored program version information 702, and a stored program 703, which are associated with each other. The stored ID 701 is information specific to the type and an individual programmable device 71 of the endoscope device 10. The stored program version information 702 is information indicating the version of the stored program 703. The stored program 703 is a program that includes code that defines the circuit configuration of the programmable device 71.

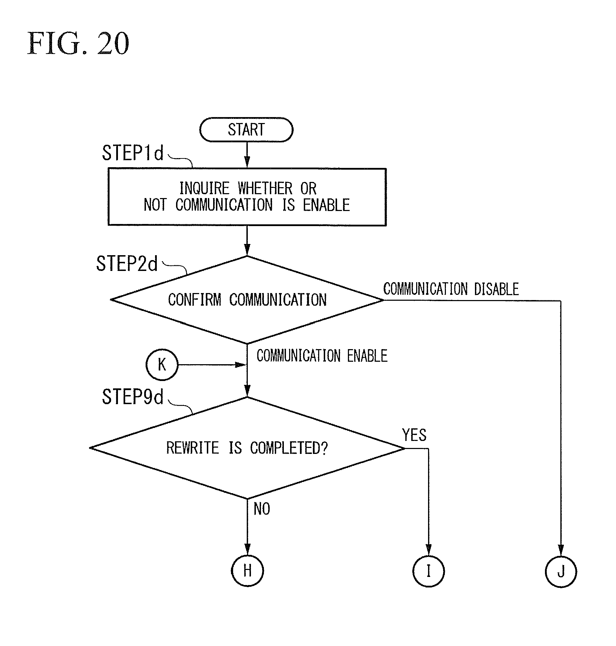

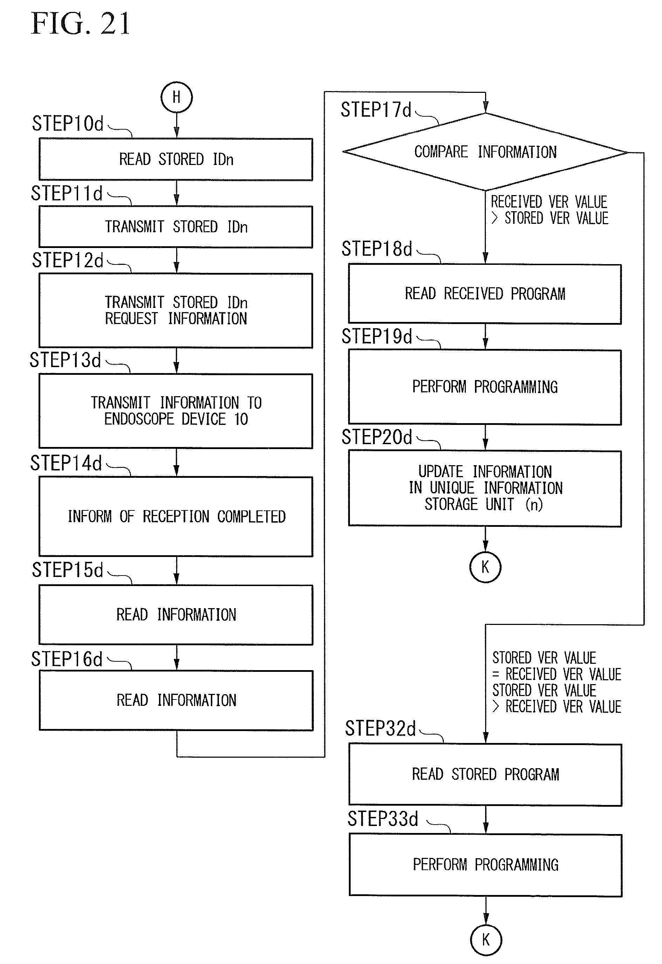

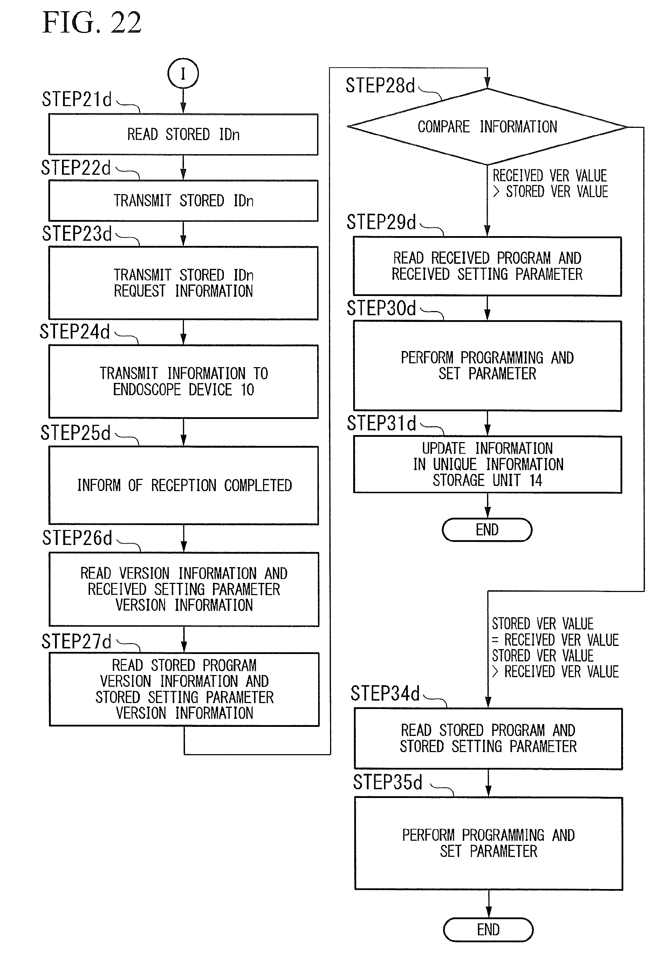

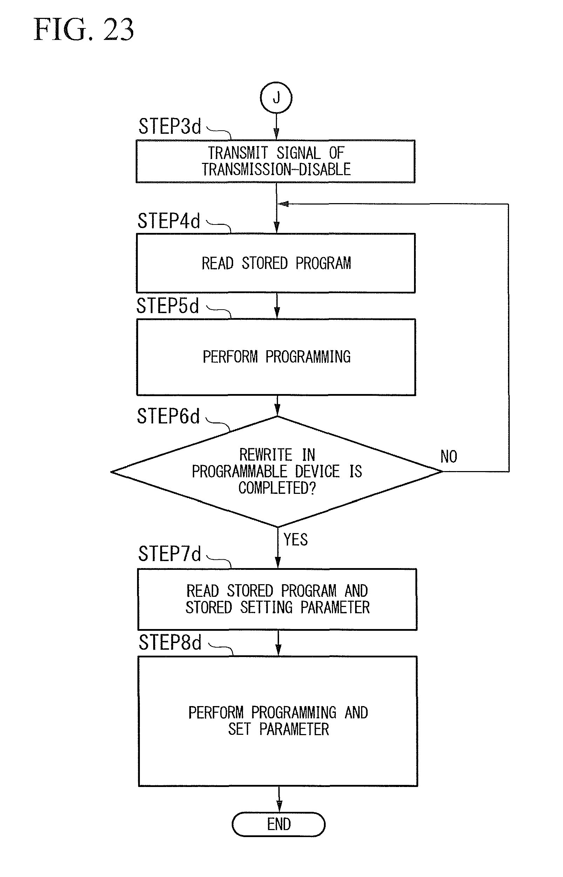

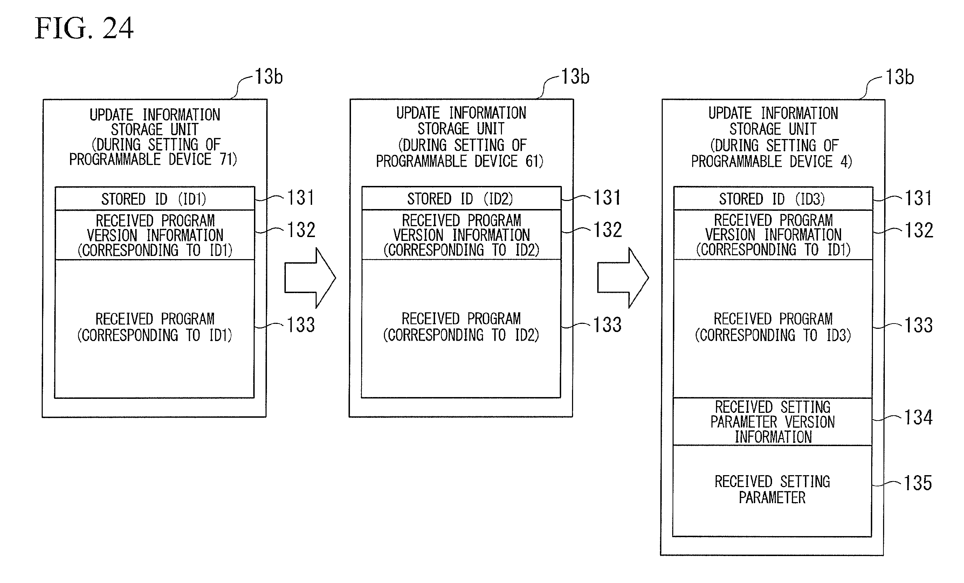

Next, the operation of the endoscope device 10 of the present embodiment will be described. FIGS. 20, 21, 22, and 23 show the procedure of operation of the endoscope device 10. Further, FIG. 24 shows temporal changes of the data stored in the update information storage unit 13b. Hereinafter, the operation of the endoscope device 10 will be described with reference to FIGS. 20 to 24. In the present embodiment, after programming for the programmable device 61 and the programmable devices 71 is performed, programming and setting of the setting parameter for the programmable device 4 are performed. In the following description, the programmable device 71 is referred to as a programmable device (1), the programmable device 61 is referred to as a programmable device (2), the programmable device 4 is referred to as a programmable device (3), the unique information storage unit 72 is referred to as a unique information storage unit (1), the unique information storage unit 62 is referred to as a unique information storage unit (2), and the unique information storage unit 14 is referred to as a unique information storage unit (3). In the following description, the initial value of n is 1.

### (STEP 1d)

The program setting unit 12 requests to the update information management unit 13 the determination of whether or not it is possible to communicate with the external storage device 20.

### (STEP 2d)

Description will be omitted because the same processing is performed as the processing performed in STEP 3 shown in the first preferred embodiment. As in the first preferred embodiment, the processing will branch as follows according to the result of the comparison by the communication unit 13a of the update information management unit 13. If the communication unit 13a of the update information management unit 13 determines that the communication with the external storage device 20 is impossible, then the processing of STEPS 3d to 8d is performed.

### (STEP 3d)

The communication unit 13a of the update information management unit 13 transmits the signal of transmission-disable to the program setting unit 12.

### (STEP 4d)

If receiving the signal of transmission-disable, then the program setting unit 12 reads the stored program from the unique information storage unit (n).

### (STEP 5d)

The program setting unit 12 performs programming for the programmable device (n) by using the stored program that has been read.

### (STEP 6d)

The program setting unit 12 recognizes the number (N) of the programmable devices mounted on the peripheral device connected to the processor unit 1. Further, the program setting unit 12 stores the number (n) of times of rewriting the programmable device, compares all of the number of programmable devices (N) with the number (n) of rewriting, and repeats the processing STEPS 4d to 5d until rewriting of all programmable devices has completed. That is, the program setting unit 12 adds 1 to n whenever rewriting the programmable device, and repeats the processing of STEPS 4d to 5d until N = n.

### (STEP 7d)

The program setting unit 12 reads the stored setting parameters 145 and stored program 143 from the unique information storage unit 14.

### (STEP 8d)

The program setting unit 12 performs programming and setting of parameters for the programmable device 4 by using the stored program 143 and the stored setting parameter 145 that have been read.

As described above, all processing of rewriting of the programmable devices is completed.

The operation when it is determined that the communication unit 13a of the update information management unit 13 can communicate with the external storage device 20 in the processing of STEP 2d is as follows.

### (STEP 9d)

The same processing as STEP 6d is performed and a description thereof will be omitted.

First, after the rewriting is performed in the order of the programmable device 71 and the programmable device 61 by the processing from STEP 10d, rewriting of the programmable device 4 is performed by the processing from STEP 21 d. In the following description, IDn is either ID1 corresponding to the programmable device 71, ID2 corresponding to the programmable device 61, or ID3 corresponding to the programmable device 4. First, the processing on rewriting of the programmable device 71 and the programmable device 61 is performed.

### (STEP 10d)

The program setting unit 12 reads the stored IDn from the unique information storage unit (n).

### (STEP 11d)

The program setting unit 12 transmits the stored IDn that has been read to the update information management unit 13.

### (STEP 12d)

The communication unit 13a of the update information management unit 13 stores the IDn received from the program setting unit 12 temporarily as the ID 131 on the update information storage unit 13b. The communication unit 13a of the update information management unit 13 transmits the stored ID 131 that has been stored to the external storage device 20, and requests the latest program and the latest program version information corresponding to the stored ID 131 that has been stored.

### (STEP 13d)

The communication unit 20a of the external storage device 20 transmits the stored ID 131 that has been received to the control unit 20b. The control unit 20b specifies the type and the programmable device from the stored ID 131. The control unit 20b reads the latest program and the latest version information corresponding to the type and the programmable device that have been specified from the information storage unit 20c, and transmits them to the endoscope device 10 via the communication unit 20a.

### (STEP 14d)

The communication unit 13a of the update information management unit 13 temporarily stores the received program as the received program 133 and the received program version information as the received program version information 132 in the update information storage unit 13b. Thereafter, the communication unit 13a of the update information management unit 13 notifies the program setting unit 12 that the reception from the external storage device 20 is completed.

### (STEP 15d)

If receiving the notification of reception completion, then the program setting unit 12 reads the received program version information 132 from the update information storage unit 13b of the update information management unit 13.

### (STEP 16d)

The program setting unit 12 reads the stored program version information from the unique information storage unit (n).

### (STEP 17d)

The program setting unit 12 compares the received program version information 132 read from the update information storage unit 13b of the update information management unit 13, with the stored program version information read from the unique information storage unit (n), and determines whether or not each information coincides with each other.

Depending on the result of the comparison by the program setting unit 12, the processing will branch as follows. If both program version information coincide with each other, or the program version information read from the unique information storage unit (n) is determined to be newer than the program version information read from the update information storage unit 13b of the update information management unit 13, then the processing of STEPS 32d to 33d is performed using the program stored in the unique information storage unit (n). Description is omitted because the processing of STEPS 32d to 33d is the same as the processing of STEPS 4d to 5d. After the end of the processing of STEP 33d, 1 is added to n. Further, if it is determined that the version information read from the update information storage unit 13b of the update information management unit 13 is newer than the version information read from the unique information storage unit (n), the processing of STEPS 18d to 20d is performed.

### (STEP 18d)

If it is determined that the value of the received program version information 132 does not coincide with the value of the stored program version information (the value of the received program version information 132 is greater than the value of the stored program version information), then the program setting unit 12 reads the receive program 133 from the update information storage unit 13b of the update information management unit 13.

### (STEP 19d)

The program setting unit 12 performs programming for programmable device (n) by using the received program 133 that has been read.

### (STEP 20d)

The program setting unit 12 reads the received program version information 132 and the received program 133 from the update information storage unit 13b of the update information management unit 13, and updates (overwrites) corresponding stored program version information and stored program stored in the unique information storage unit (n). After updating is completed, 1 is added to n, and the processing of STEP 9d is performed again.

If the processing on rewriting of the programmable device 71 and the programmable device 61 is completed, then the processing on rewriting of the programmable device 4 is performed.

### (STEPS 21 d to 31d, STEPS 34d to 35d)

The same processing is performed as STEPS 1 to 2 and 5 to 15 shown in the first preferred embodiment, and description thereof will be omitted.

In the processing described above, as shown in FIG. 24, information for rewriting of the programmable device 71 is initially stored in the update information storage unit 13b. Subsequently, information for rewriting of the programmable device 61 is stored, and information for rewriting the programmable device 4 is stored last.

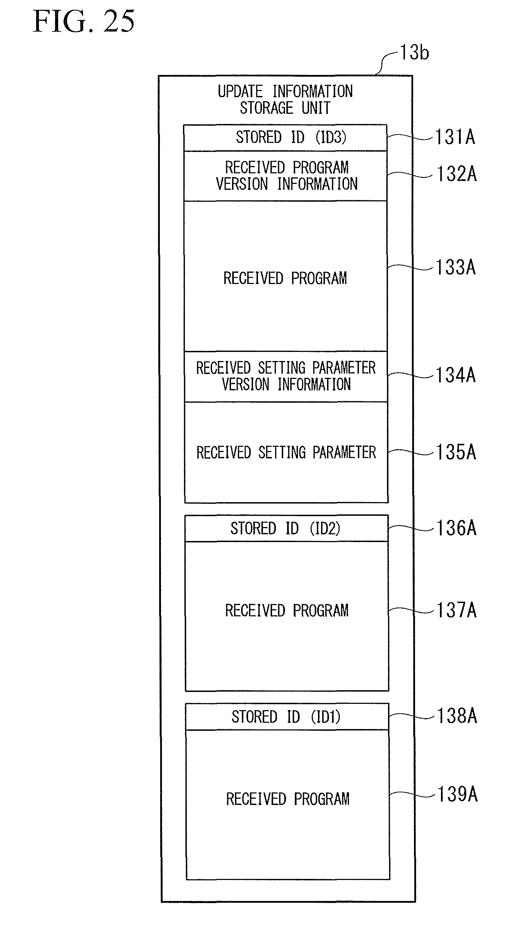

In the present embodiment, it is described that the procedure is repeated from reading of the ID until programming for each programmable device. However, it is possible that the update information management unit 13 temporarily stores the ID of all programmable devices and transmits the ID together to the external storage device 20. FIG. 25 shows the structure of data stored in the update information storage unit 13b of this case. The update information storage unit 13b stores the stored ID 131A (ID 3) corresponding to the programmable device 4, the received program version information 132A, the received program 133A, the received setting parameter version information 134A, and the received setting parameter 135, which are associated with each other. Also, the update information storage unit 13b stores the stored ID 136A (ID 2) corresponding to the programmable device 61 and the received program 137A, which are associated with each other. Also, the update information storage unit 13b stores the stored ID 138A (ID 1) corresponding to the programmable device 71 and the received program 139A, which are associated with each other.

As described above, by rewriting each programmable device of the image capturing unit 6 and the light source unit 7 connected to the processor unit 1 into the latest program, it is possible to keep the state of the programmable device in the latest state.

In the present embodiment, different ID is used for each type and programmable device of the endoscope device. Because the peripheral device of the processor unit 1 may be changed in accordance with the intended use, it can be recognized from the ID which peripheral device is connected during the use. Furthermore, it can be recognized from the program version information whether a program stored in the unique information storage unit of the peripheral device is the latest program.

In the present embodiment, an example is described in which the programmable device is mounted on two peripheral devices of the processor unit 1. The programmable device may be mounted on more than two peripheral devices. Further, only the setting parameter of the programmable device 4 is used in the present embodiment. However, it may be possible to use the setting parameter individually for other programmable devices in associated with the ID and the program. Further, it is also possible to perform various modifications described in the first preferred embodiment.

### (Sixth Preferred Embodiment)

Next, a sixth preferred embodiment of the present invention will be described. The configuration of the present embodiment is the same as the configuration of the first preferred embodiment. In the present embodiment, the external storage device 20 is configured as a memory device, for example. The program setting unit 12 retrieves and reads the latest version information used for the comparison of the version information from the external storage device 20. In the present embodiment, the program setting unit 12 acquires the version information corresponding to the ID read from the unique information storage unit 14 by referring to all ID and all version information stored in the external storage device 20 through the update information management unit 13, which procedure will be described. In the present embodiment, with respect to those having the same functions as those in the first preferred embodiment, description thereof is omitted for the same reference numerals.

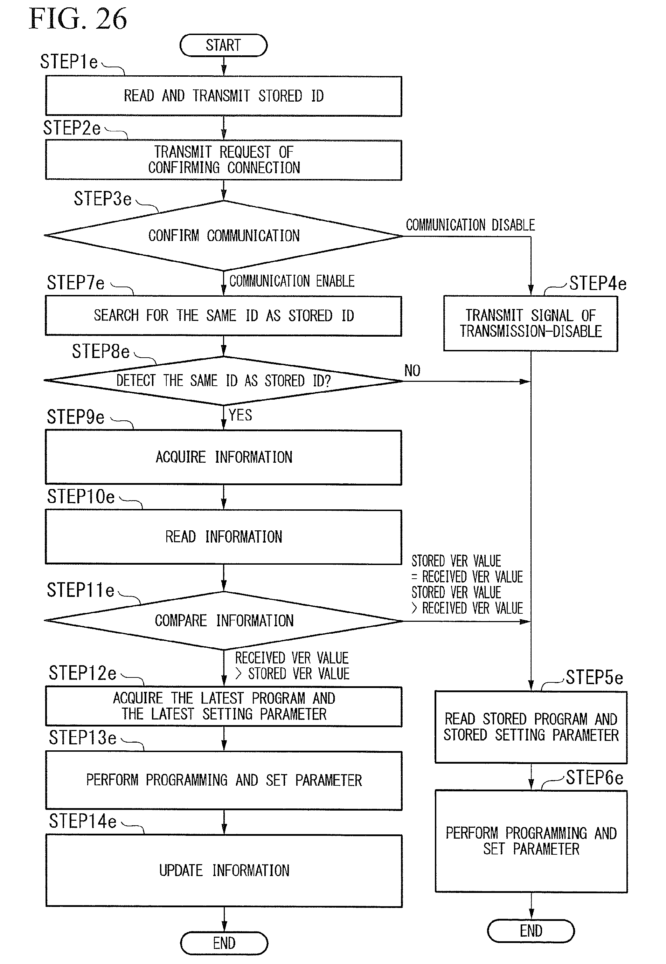

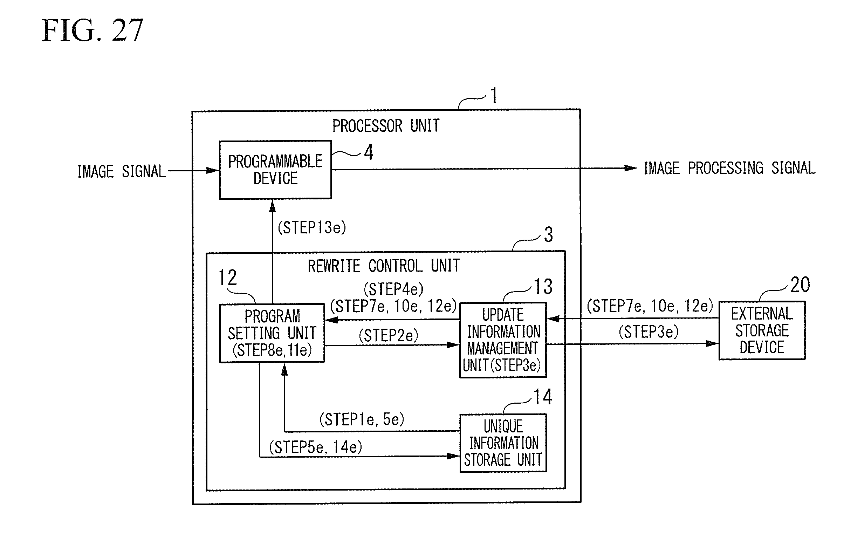

Next, the operation of the endoscope device 10 of the present embodiment will be described. FIG. 26 shows the procedure of the operation of the endoscope device 10. FIG 27 shows the relationship between each step of FIG. 26 and the configuration related to the operation of the present embodiment of the configuration shown in FIG. 1. Hereinafter, a description will be given of the operation of the endoscope device 10 with reference to FIGS. 26 to 27.

### (STEP 1e)

The program setting unit 12 reads the stored ID 141 from the unique information storage unit 14, and transmits the stored ID 141 that has been read to the update information management unit 13.

### (STEP 2e)

The program setting unit 12 requests to update information management unit 13 confirmation of connection with the external storage device 20.

### (STEP 3e)

If the communication unit 13a of the update information management unit 13 receives a request, then determines whether or not the communication with the external storage device 20 is possible.

Depending on the result of the determination of the communication unit 13a of the update information management unit 13, the processing will branch as follows. If the communication with the external storage device 20 is not possible, then the processing of STEPS 4e to 6e is performed.

### (STEPS 4e to 6e)

Description is omitted because the same processing is performed as STEPS 4 to 6 shown in the first preferred embodiment.

If the communication unit 13a of the update information management unit 13 determines that it is possible to communicate with the external storage device 20, then the processing of STEPS 7e to 14e is performed.

### (STEP 7e)

The program setting unit 12 reads a plurality of IDs stored in the external storage device 20 via the update information management unit 13, and confirms whether or not there is an ID that coincides with the stored ID 141. The information of the ID or the like stored in the external storage device 20 is stored in the area of the storage medium designated in advance. The program setting unit 12 sequentially reads each ID by specifying the region, and compares them with the stored ID141.

### (STEP 8e)

The program setting unit 12 determines whether or not an ID that coincides with the stored ID 141 can be acquired.

Depending on the result of the comparison by the program setting unit 12, the processing will branch as follows. If all ID stored in the external storage device 20 does not coincide with the stored ID 141, then it is determined that the program and the setting parameter corresponding to the stored ID 141 is not stored in the external storage device 20, and the processing of STEPS 5e to 6e is performed. Further, if any ID stored in the external storage device 20 coincides with the stored ID 141, then the processing of STEPS 9e to 14e is performed.

### (STEP 9e)

The program setting unit 12 reads from the external storage device 20 the latest program version information and the latest setting parameter version information corresponding to the ID that is read from the external storage device 20.

### (STEP 10e)

The program setting unit 12 reads the stored program version information 142 and the stored setting parameter version information 145 from the unique information storage unit 14.

### (STEP 11e)

The program setting unit 12 compares the program version information read from the external storage device 20 with the stored program version information 142 read from the unique information storage unit 14. At the same time, the program setting unit 12 compares the setting parameter version information read from the external storage device 20 with the stored setting parameter version information 145 read from the unique information storage unit 14, to confirm whether or not each information corresponds to each other.

Depending on the result of the comparison by the program setting unit 12, the processing will branch as follows. If both version information coincide with each other, or if it is determined that the version information read from the unique information storage unit 14 is newer than the version information read from the external storage device 20, then the processing of STEPS 5e to 6e is performed, and rewriting process is completed. Further, if it is determined that the version information read from the external storage device 20 is newer than the version information read from the unique information storage unit 14, then the processing of STEPS 12e to 14e is performed.

### (STEP 12e)

If it is determined that the value of the program version information and the setting parameter version information read from the external storage device 20 does not coincide with the value of the stored program version information 142 and the stored setting parameter version information 145 (the value of the program version information and the setting parameter version information read from the external storage device 20 are larger than the value of the stored program version information 142 and the stored setting parameter version information 145), then the program setting unit 12 reads from the external storage device 20 the latest program and the latest setting parameter corresponding to the stored ID 141.

### (STEP 13e)

By using the program and the setting parameter that have been read, the program setting unit 12 performs programming and setting of parameter for the programmable devices 4.

### (STEP 14e)

The program setting unit 12 updates (overwrites) the information of each corresponding program version information 142, stored program 143, stored setting parameter version information 144, and stored setting parameter 145 that are stored in the unique information storage unit 14 by using the program version information, the program, the setting parameter version information, and the setting parameter that are read from the external storage device 20. After update is completed, rewriting processing to the programmable device 4 is completed.

As described above, since the program setting unit 12 acquires the information directly from the external storage device 20, it is possible to make the external storage device 20 have a simple structure such as a memory device or the like. Assuming that the external storage device 20 is the memory device, the program setting unit 12 or the update information management unit 13 generates an input/output signal that suits with the specification of the memory device.

Various modifications are possible in the present embodiment. For example, as in the first preferred embodiment, the program setting unit 12 first reads the program and the setting parameter from the external storage device 20, and updates (overwrites) the stored program 143 and the stored setting parameters 145 of the unique information storage unit 14. Then the stored program 143 and the stored setting parameter 145 are read from the unique information storage unit 14, and are installed on the programmable device 4. The above method is also possible. It is also possible to perform various modifications described in the first preferred embodiment besides this.

While preferred embodiments of the invention have been described and illustrated above, it should be understood that these are exemplary of the invention and are not to be considered as limiting. Additions, omissions, substitutions, and other modifications can be made without departing from the spirit or scope of the present invention.

### INDUSTRIAL APPLICABILITY

The present invention can be applied to the endoscope device and an endoscope system that can install on the programmable device the programs corresponding to each programmable device. The endoscope device transmits to an external storage device storing a plurality of programs corresponding to a plurality of programmable devices, unique information specific to the programmable device that the endoscope device itself has, and receives from the external storage device a program of the programmable device corresponding to the unique information. Thereby, the endoscope device can install on the programmable device a program corresponding to each programmable device.

### DESCRIPTION OF THE REFERENCE SYMBOLS

1 ... processor unit, 3 ... rewrite the control unit, 4,5,61,71 ... programmable device, 6 ... image capturing unit, 7 ... light source unit, 10 ... endoscope device , 12 ... program setting unit, 13 ... update information management unit, 13a, 20a ... communication unit, 13b ... update information storage unit, 14,62,72 ... unique information storage unit, 20 ... external storage device, 20b ... control unit, 20c ... information storage unit, 63 ... image capturing device, 73 ... light source

## Claims

1. An endoscope device that communicates with an external storage device, the external storage device storing a plurality of programs corresponding to a plurality of programmable devices, the endoscope device comprising:
a programmable device that performs a process in accordance with a program; and
a rewrite control unit that transmits to the external storage device unique information specific to the programmable device, the rewrite control unit receiving from the external storage device a program corresponding to the unique information, the rewrite control unit installing the program, which has been received, on the programmable device.

2. The endoscope device according to claim 1, wherein
the unique information is a type and an ID of the programmable device, and
the rewrite control unit includes a unique information storage unit having a nonvolatile storage medium that stores the ID.

3. The endoscope device according to claim 2, wherein
the rewrite control unit further includes a communication unit that transmits to the external storage device the ID that has been stored in the unique information storage unit.

4. The endoscope device according to claim 3, wherein
the communication unit further receives from the external storage device the program corresponding to the ID, and
the rewrite control unit further includes an update information storage unit having a storage medium that stores the program that has been received from the external storage device.

5. The endoscope device according to claim 4, wherein
the rewrite control unit further includes a program setting unit that reads the program, which corresponds to the ID that has been stored in the unique information storage unit, from the update information storage unit, the program setting unit installing the program, which has been read, on the programmable device.

6. The endoscope device according to claim 5, wherein
the communication unit further receives version information of the program from the external storage device,
the unique information storage unit stores the program and the version information, which have been installed on the programmable device, the program and the version information being associated with the ID, and
the update information storage unit stores the program and the version information, which have been received, the program and the version information being associated with the ID.

7. The endoscope device according to claim 6, wherein
the program setting unit compares the version information, which has been stored in the unique information storage unit, and the version information, which has been stored in the update information storage unit, that are associated with a same ID,
reads from the update information storage unit or the unique information storage unit the program associated with a newer version information, and
installs the program, which has been read, on the programmable device.

8. The endoscope device according to claim 7, wherein,
if the version information stored in the update information storage unit is newer than the version information stored in the unique information storage unit, the program setting unit further rewrites the program and the version information, which have been stored in the unique information storage unit, into the program and the version information that have been stored in the update information storage unit.

9. The endoscope device according to claim 5, wherein,
if the communication unit is disconnected from the external storage device, the program setting unit further reads the program from the unique information storage unit and installs the program, which has been read, on the programmable device.

10. The endoscope device according to claim 5, wherein
the communication unit receives the program, which corresponds to the ID, and the parameter information, which is specific to the programmable device, from the external storage device,
the update information storage unit stores the program and the parameter information, which have been received from the external storage device, the program and the parameter information being associated with the ID,
the program setting unit reads the program, which corresponds to the ID that is stored in the unique information storage unit, from the update information storage unit,
installs the program, which has been read, on the programmable device,
reads the parameter information, which corresponds to the ID and stored in the unique information storage unit, from the update information storage unit, and
installs the parameter information, which has been read, on the programmable device.

11. The endoscope device according to claim 6, comprising a plurality of the programmable device,
wherein the unique information storage unit stores the program and the version information, the program and the version information being associated with the ID, for each of the plurality of the programmable devices.

12. The endoscope system according to claim 5, wherein
the communication unit further transmits to the external storage device the ID and the version information of the program corresponding to the ID.

13. An endoscope system including an external storage device, which holds a plurality of programs corresponding to the plurality of programmable devices, and an endoscope device, wherein
the endoscope device comprises:
a programmable device that performs a process according to the program; and
a rewrite control unit that transmits a type of the programmable device, an ID of the programmable device and the version information corresponding to the ID, receives the program corresponding to the ID from the external storage device, and installs the program, which has been received, on the programmable device,
the rewrite control unit comprises:
a unique information storage unit having a nonvolatile storage medium that stores the ID;
a first communication unit that transmits to the external storage device the ID, which has been stored in the unique information storage unit, and the version information of the program corresponding to the ID, the first communication unit receiving the program corresponding to the ID from the external storage device;
an update information storage unit having a storage medium that stores the program that has been received from the external storage device; and
a program setting unit that reads from the update information storage unit the program corresponding to the ID that has been stored in the unique information storage unit, the program setting unit installing the program, which has been read, on the programmable device, and
the external storage device comprises:
an information storage unit having a storage medium that stores the program and the version information, the program and the version information being associated with the ID;
a second communication unit that receives the ID and the version information from the endoscope device, the second communication unit transmitting to the endoscope device the program, which corresponds to the ID that has been received from endoscope device, of the program that has been stored in the information storage unit; and
a control unit that reads from the information storage unit the version information, which corresponds to the ID that has been received from the endoscope device, so as to compare with the version information, which has been received from the endoscope device, the control unit determining whether or not to transmit the program, which has been stored in the information storage unit, to the endoscope device based on a result of the comparing.
